(19) Europäisches Patentamt
European Patent Office
Office européen des brevets

(11) Publication number: **0 109 044**
**B1**

(12) **EUROPEAN PATENT SPECIFICATION**

(45) Date of publication of patent specification: **15.10.86**

(21) Application number: **83111182.8**

(22) Date of filing: **09.11.83**

(51) Int. Cl.$^4$: **C 07 D 499/00,** A 61 K 31/43
// C07D205/08, C07F9/65,
C07D211/62, C07D211/60

(54) **Penems, pharmaceutical compositions containing them, process for preparing them.**

(30) Priority: **16.11.82 US 441989**
**16.11.82 US 441988**
**06.12.82 US 446928**

(43) Date of publication of application:
**23.05.84 Bulletin 84/21**

(45) Publication of the grant of the patent:
**15.10.86 Bulletin 86/42**

(84) Designated Contracting States:
**AT BE CH DE FR GB IT LI NL SE**

(56) References cited:
**EP-A-0 002 210**
**EP-A-0 003 960**
**EP-A-0 058 317**
**EP-A-0 070 204**
**GB-A-2 042 515**

**The file contains technical information submitted after the application was filed and not included in this specification**

(73) Proprietor: **SCHERING CORPORATION**
**2000 Galloping Hill Road**
**Kenilworth, New Jersey 07033 (US)**

(72) Inventor: **Afonso, Adriano**
**10 Woodmere Road**
**West Caldwell New Jersey 07006 (US)**
Inventor: **Weinstein, Jay**
**191 Bellevue Avenue**
**Upper Montclair New Jersey 07043 (US)**
Inventor: **Ganguly, Ashit Kumar**
**96 Cooper Avenue**
**Upper Montclair New Jersey 07043 (US)**
Inventor: **Girijavallabhan, Viyyoor Moopil**
**10 Maplewood Drive**
**Parsippany New Jersey 07054 (US)**

(74) Representative: **Ritter, Stephen David et al**
**Mathys & Squire 10 Fleet Street**
**London EC4Y 1AY (GB)**

**EP 0 109 044 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to chemical compounds of the kind known as penems, in particular 6-substituted-2-substituted penems, to processes for their preparation, and pharmaceutical compositions containing them.

Some penem compounds are known, for example from European Patent Specifications publication Nos. 0 003960, 0 013662 and 0 002210 and from GB—A—2 042 515.

The present invention provides novel penem compounds having the general formula

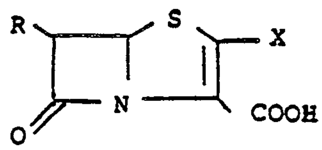

I

wherein R is hydroxy-loweralkyl, and X is

(i) (a) 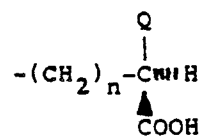 or

(b) 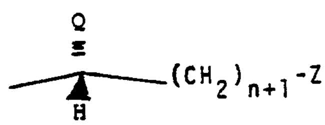

in which n is 0 to 4; Q is $—NR_1R_2$ or

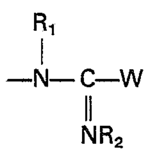

in which $R_1$ and $R_2$ are independantly chosen from hydrogen, loweralkyl, loweralkenyl, heteroaryl, phenyl, phenyl substituted by one or more groups chosen from chloro, bromo, fluoro, loweralkyl, hydroxy, nitro, amino, aminomethyl, mono-loweralkylamino, di-loweralkylamino, loweralkoxy and carboxy, or $R_1$ is hydrogen and $R_2$ is acyl; W is chosen from hydrogen, loweralkyl or amino; and Z is hydrogen, 4-imidazolyl, 3-indolyl, phenyl, p-hydroxyphenyl, branched lower alkyl, $—COOR_1$, $—OR_1$, $—NR_1R_2$ or $—SR_1$ wherein $R_1$ and $R_2$ are as defined above; or

(ii) 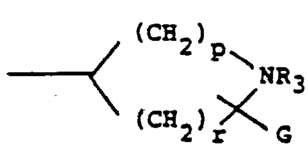

wherein p is 2 to 6, r is 0, 1, 2 or 3, $R_3$ is hydrogen, loweralkyl, or

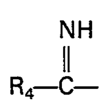

in which $R_4$ is hydrogen or $C_1—C_3$ alkyl; and G is hydrogen, loweralkyl, loweralkoxy, amino, hydroxy, carboxy, or lowralkylthio, and the pharmaceutically acceptable salts and metabolisable esters thereof.

Preferred compounds having the part structure (i)(a) above are those having the formula II

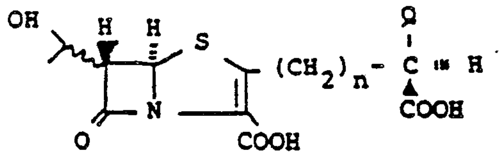

II

where *n* and Q are as defined above, and the pharmaceutically acceptable salts and esters thereof. The more preferred of these compounds are those having the formula IIa

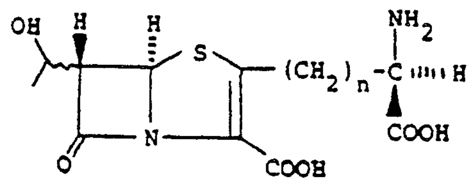

II(a)

and the pharmaceutically acceptable salts and esters thereof. Also preferred as category (i)(a) compounds are those of formula II in which *n* is 2, especially having a 3′-amino-3′-carboxypropyl group as the 2-substituent.

The preferred compounds having the part structure (i)(b) are those represented by formula III

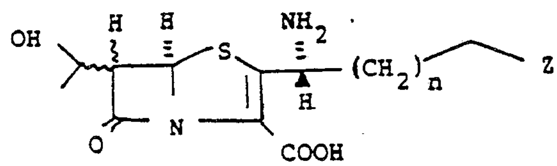

III

in which n is as defined above, and Z is chosen from hydrogen, carboxy, hydroxy, loweralkylthio or amino, and the pharmaceutically acceptable salts and esters thereof.

More preferred compounds of part structure (i)(b) are those of formula III′

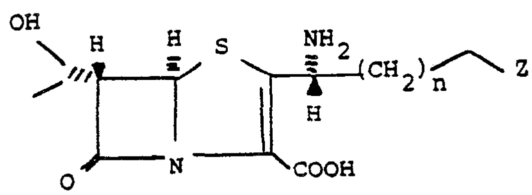

III′

in which *n* and Z are as defined above.

A particularly preferred group of compounds of formula 1(i)(b) (erspecially including compounds of formulae III and III′) are those in which *n* is 1 and Z is lower alkylthio or carboxy.

Preferred compounds of category (ii) are those wherein (r + p) equals 4. Also preferred are those wherein G is hydrogen or methyl. Highly preferred compounds of this category are those in which (r + p) equals 4 *and* G is hydrogen or methyl, especially those in which $R_3$ is hydrogen, acetimidoyl or amidino.

The lower alkyl groups referred to above contain 1 to 6 carbon atoms and include methyl, ethyl, propyl, butyl, pentyl, hexyl and the corresponding branched chain isomers thereof.

The lower alkenyl groups referred to above contain 2 to 6, and preferably 2 to 4, carbon atoms, and are, for example, vinyl, allyl, but-2-enyl or but-3-enyl groups.

The lower alkoxy groups referred to above contain 1 to 6, and preferably 1 to 3, carbon atoms and are illustrated by methoxy, ethoxy, *n*-propoxy, isopropoxy and the like.

The term "heteroaryl" as used herein refers to a heterocyclic group of aromatic character which contains 5 to 7 ring atoms of which 3 to 6 are carbon atoms and the remaining ring atoms are nitrogen, sulfur or oxygen atoms. Typical heteroaryl groups are those such as pyridyl, for example, pyrid-2-yl, pyrid-3-yl or pyrid-4-yl, thienyl, for example, thien-2-yl, or furyl, for example, fur-2-yl.

The acyl groups referred to above contain 2 to 18 carbon atoms and are the residue of a sulfonic acid or a carboxylic acid, such as acetyl, propionyl, valeryl and butyryl.

The compounds of the present invention possess at least two asymetric carbon atoms or chiral centres, indicated in the partial formula below at the 5 and 6-position carbon atoms.

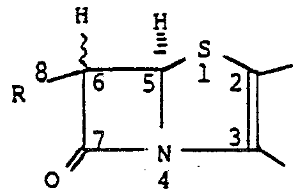

The configuration at the 5 position is R. The overall configurations at these centers may thus be 5R, 6R, and 5R, 6S. The preferred configuration is 5R, 6S.

Additionally there may be an asymetric carbon atom or chiral centre in the hydroxyloweralkyl group "R", in which case such centre preferably has the R configuration. As 1-hydroxyethyl is the preferred "R" group, the preferred configuration at the three centres discussed above will be 5R, 6S, 8R.

The compounds of category (i)(a) and (i)(b) possess at least 3 asymetric carbon atoms, i.e. with one, in addition to those shown above, in the side chain to which the nitrogen atom of group Q is attached. The stereochemistry for this carbon atom in the category (i)(a) compounds is R when *n* is 1 to 4 and S when *n* is O. However the use of the diastereomeric form at the amino acid carbon is also contemplated and thus the present invention includes both the diastereomeric mixture (at the amino acid carbon) as well as the individual diastereoisomer.

The stereochemistry of the corresponding carbon atom in the category (i) (b) compounds, namely the 1′ position carbon atom, is R, as shown in the drawing of he 2-side chain, and the present invention contemplates not only the pure isomer (that is the designated isomer in the absence of other isomers of the same compound) but mixtures of the designated isomer with other stereoisomers of the same compound.

In the category (ii) compounds, depending upon the particular values for p, r and G, one or more asymetric carbon atoms may exist in the ring substituent attached to the 2-position. The present invention contemplates individual isomers as well as enantiomeric and diastereomeric mixtures of these compounds.

Pharmaceutically acceptable salts in accordance with the invention include salts formed from organic or inorganic bases, as well as acid addition salts and internal salts i.e. zwitterions.

suitable pharmaceutically acceptable salts include the alkali metal, alkaline earth metal, amine and acid addition salts, for example sodium, potassium, aluminium, magnesium and calcium salts, and amine salts which may be formed from a wide variety of suitable organic amines, such as aliphatic, or aliphatic primary, secondary or tertiary mono-, di- or polyamines, or heterocyclic bases, for example from triethylamine, 2-hydroxyethylamine, di-(2-hydroxyethyl)amine, tri-(2-hydroxyethyl)amine, 4-aminobenzoic acid 2-diethylaminoethyl ester, 1-ethylpiperidine, bicyclohexylamine, N,N′-dibenzylethylenediamine, pyridine, collidine, quinoline, procaine, dibenzylamine, 1-ephenamine and N-alkylpiperidine. Typical acid addition salts are those formed with mineral acids such as hydrochloric, hydrobromic, hydroiodic, phosphoric and sulfuric or with suitable carboxylic acids or sulfonic acids such as trifluoroacetic, *p*-toluenesulfonic, maleic, acetic, citric, oxalic, succinic, benzoic, tartaric, fumaric, mandelic, ascorbic, lactobionic and malic.

The metabolizable esters are the physiologically cleavable esters, *i.e.,* those esters known in the penicillin, cephalosporin and penem arts to be easily cleaved within the body to the parent acid. Examples of such metabolizable esters are those such as indanyl, phthalidyl, methoxymethyl, glycyloxymethyl, phenylglycyloxymethyl, thienylglycyloxymethyl or acyloxymethyl of the formula

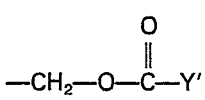

wherein Y′ is lower alkyl or phenyl. Particularly preferred metabolizable esters are methoxymethyl, acetoxymethyl, pivaloyloxymethyl, phthalidyl and indanyl.

Preparation of these salts and metabolizable esters may be carried out according to conventional procedures for forming salts and esters of beta-lactam antibiotics such as penicillins and cephalosporins.

Representative examples of compounds of this invention include the following:

1) (5R,6S,8R,1′R)-6-(1-hydroxyethyl)-2-(1′-aminoethyl)-2-penem-3-carboxylic acid,

2) (5R,6S,8R,1′R)-6-(1-hydroxyethyl)-2-[(1′-amino-3′-carboxy)-propyl]-2-penem-3-carboxylic acid,

3) (5R,6S,8R,1′R)-6-(1-hydroxyethyl)-2-[(1′-amino-3′-methylthio)propyl]-2-penem-3-carboxylic acid,

4) (5R,6S,8R,1′R)-6-(1-hydroxyethyl)-2-[(1′-amino-2′-methylthio)ethyl]-2-penem-3-carboxylic acid,

5) (5R,6S,8R,1′R)-6-(1-hydroxyethyl)-2-[1′,5′-diaminopentyl)]-2-penem-3-carboxylic acid,

6) sodium (5R,6S,8R,1′R)-6-(1-hydroxyethyl)-2-[(1′-acetimidoyl-3′-methylthio)propyl]-2-penem-3-carboxylate or the free acid,

7) sodium (5R,6S,8R,1'R)-6-(1-hydroxyethyl)-2-[(1'-guanidino-3'-methylthio)propyl]-2-penem-3-carboxylate or the free acid,

8) (5R,6S,8R,2'R)-6-(1-hydroxyethyl)-2-(2'-amino-2'-carboxyethyl)-2-penem-3-carboxylic acid,

9) (5R,6S,8R,4'R)-6-(1-hydroxyethyl)-2-(4'-amino-4'-carboxy)-butyl-2-penem-3-carboxylic acid,

10) (5R,6S,8R)-6-(1-hydroxyethyl)-2-(4'-piperidinyl)-2-penem-3-carboxylic acid,

11) (5R,6S,8R,3'RS)-6-(1-hydroxyethyl)-2-(3'-piperidinyl)-2-penem-3-carboxylic acid,

12) (5R,6S,8R,2'RS)-6-(1-hydroxyethyl)-2-(2'-piperidinyl)-2-penem-3-carboxylic acid,

13) (5R,6S,8R,2'R)-6-(1-hydroxyethyl)-2-(2'-pyrrolidinyl)-2-penem-3-carboxylic acid,

14) (5R,6S,8R)-6-(1-hydroxyethyl)-2-(N-acetimidoyl-4'-piperidinyl)-2-penem-3-carboxylic acid,

15) (5R,6S,8R)-6-(1-hydroxyethyl)-2-(N-amidino-4'-piperidinyl)-2-penem-3-carboxylic acid,

and the pharmaceutically acceptable salts and metabolisable esters of the foregoing free acids.

The compounds of this invention possess antibacterial activity which may be determined by testing in standardized *in vitro* dilution tests for minimum inhibitory concentrations (MICs). Using such standard microbiological procedures, the compounds of this invention are found to exhibit activity against gram-positive and gram-negative bacteria such as *Staphylococcus aureus, Escherichia coli* and *Pseudomonas aeruginosa* at test levels of 0.1 to 100 mcg/ml. Additionally, they show activity against such organisms in the presence of penicillinase and cephalosporinase, indicating a resistance to these enzymes.

As antibacterial agents, the compounds of the present invention can be formulated in conventional manner for oral, parenteral and topical use. Thus the instant invention includes within its scope pharmaceutical compositions comprising the novel compounds of the invention in admixture with a pharmaceutically acceptable carrier or excipient therefor.

The dosage administered of the penems of this invention is dependent upon a variety of factors *i.e.,* the age and weight of the individual being treated, the mode of administration, and the type and severity of the bacterial infection being prevented or reduced. Typically the dosage administered per day is 1 to 250 mg/kg and preferably from about 5 to 20 mg/kg in divided dosages. Typically, the dosage will be administered in dosage units containing convenient amounts, for example, 125, 250 or 500 mg of active ingredient combined with a suitable physiologically acceptable carrier or diluent.

For oral administration, the compounds of this invention are typically formulated as tablets, capsules or elixirs. For parenteral administration they may be formulated into solutions or suspensions. Typical topical formulations are lotions, creams, ointments, sprays, and mechanical delivery systems, e.g. transdermal.

Typical pharmaceutically acceptable carriers for use in the formulations described above are exemplified by: sugars such as lactose, sucrose, mannitol and sorbitol; starches such as corn starch, tapioca starch and potato starch; cellulose and derivatives such as sodium carboxy-methyl cellulose, ethyl cellulose, and methyl cellulose; calcium phosphates such as dicalcium phosphate and tri-calcium phosphates; sodium sulfate; calcium sulfate; polyvinyl pyrrolidone; polyvinyl alcohol; stearic acid; alkaline earth metal starates such as magnesium stearate; stearic acid, vegetable oils such as peanut oil, cottonseed oil, sesame oil, olive oil and corn oil; non-ionic, cationic and anionic surfactants; ethylene glycol polymers; beta-cyclodextrin; fatty alcohols; hydrolyzed cereal solids, water; polyalkylene glycols; isopropanol; gelatin; benzyl alcohol; gums; and petrolatum; as well as other non-toxic compatible fillers, binders, disintegrants and lubricants commonly used in pharmaceutical formulations. Optionally, the compositions may also contain preservatives, aerosol propellants such as hydro-carbons; and colouring, thickening, suspending, dispersing, emulsifying, wetting, stabilizing and buffering agents. In addition, there may also be included in the composition other active ingredients to extend the spectrum of antibacterial activity and/or provide relief of concommittant symptoms such as inflammation.

The compounds of the present invention can be prepared by intramolecular cyclisation of a compound of formula IV

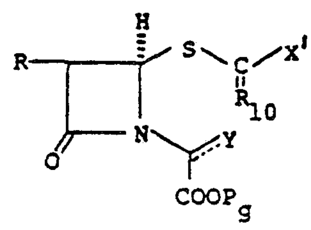

IV

in which $R_{10}$ is oxygen or sulphur, R is as defined above, Pg is a carboxy protecting group, X' represents

a) 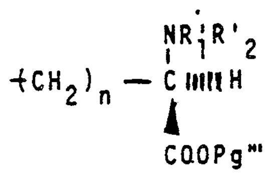

b) 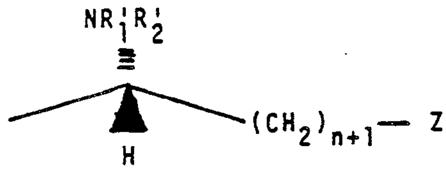

in which $R_1'$ and $R_2'$ represent respectively $R_1$ and $R_2$ as defined above except that they cannot simultaneously represent hydrogen or $R_1'$ corresponds to $R_1$ as defined above and $R_2'$ is an amino protecting group, Pg''' us a carboxy protecting group and any functional group in Z is protected if necessary or desired; or

c) 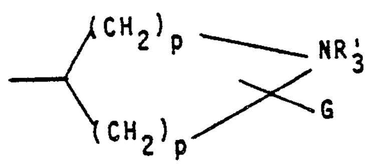

in which $R_3'$ is loweralkyl or an amino protecting group and any functional group in G is protected if necessary or desired, any other functional groups in compound IV are protected if necessary or desired and Y is a phosphonio group being double bonded to the adjacent carbon atom or a phosphonato group with a single bond to the adjacent carbon atom the negative charge of which is compensated by the presence of a cation, followed by removal of any protecting groups before or after any necessary or desired separation of stereoisomers if a mixture of stereoisomers was subjected to cyclisation, and if necessary or desired subjecting the resulting compound to one or more of the following operations:

(i) converting a free acid group to a pharmaceutically acceptable salt or metabolisable ester group;
(ii) converting a salt to a pharmaceutically acceptable metabolisable ester or free acid;
(iii) introducing the grouping

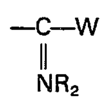

into an amine group represented by Q;
(iv) replacing the substituent $R_3'$, when it represents hydrogen, by amidine or the grouping

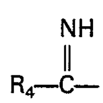 ;

(v) separation of a mixture of enantiomers.

The cyclisation of the intermediate of formula IV is preferably accomplished by dissolving the intermediate in a suitable organic solvent such as benzene, toluene or xylene and heating at reflux temperature for 24 to 48 hours.

The amino group represented by Q or $R_3$ may be converted to corresponding guanidine or amidine group e.g. where an amino group Q is converted to the

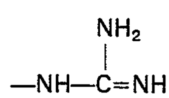

group, as described in ICAAC 11th International Congress of Chemotherapy, 19th Interscience Conference on Antimicrobial Agents and Chemotherapy, Boston, October 1—5, 1979, Papers 231 and 232 and Belgian Patent 848, 545.

Salts and esters of the compounds of formula I may be produced by methods well known in the β-lactam art. For example, salts of such compounds with acid groups can be formed by treating with metal compounds such as alkali metal salts of suitable carboxylic acids, or with ammonia or a suitable organic amine, wherein preferably a stoichiometric amount or only a small-excess of the salt-forming agent is used. Acid addition salts of the compounds of formula I with basic groupings are obtainable in the usual manner, for example, by treating with an acid or a suitable anion exchange reagent. The metabolizable esters can be prepared by reacting the corresponding alkali metal salt of formula I with a chloride of the esterifying group, preferably in a solvent such as dimethylformamide.

The protecting groups used may be any of those conventionally used in the β-lactam art. Suitable hydroxy protecting groups are those known in the art such as t-butoxycarbonyl, p-nitrobenzyloxycarbonyl and 2,2,2-trichloroethoxy-carbonyl with the latter preferred. Typical amino protecting groups are those known in the art, such as allyloxycarbonyl, 2,2,2-trichloroethoxycarbonyl, 2-bromo-ethoxycarbonyl, 4-methoxybenzyloxycarbonyl and t-butoxycarbonyl, with allyloxycarbonyl being most preferred. Typical carboxy protecting groups are allyl, 2,2,2-trichloroethyl, 2-bromoethyl, 4-methoxybenzyl and t-butyl with allyl being preferred.

The reaction conditions for any necessary deprotection depends upon the nature of the protecting groups and such conditions are well known to the skilled man.

For instance, a 2,2,2-trichloro-ethoxycarbonyl group is preferably removed by treatment with zinc and glacial acetic acid at temperatures in the range of from −30° to 0°C; groups such as *p*-nitrobenzyloxycarbonyl are removed by hydrogenolysis, for example by treating with hydrogen in the presence of a noble metal catalyst such as palladium. Allyl and allyloxycarbonyl protecting groups are most preferably removed utilizing the method described in European Patent Application publication No. 13662 which utilizes e.g. 2-ethyl-hexanoic acid or an alkali metal salt thereof and as catalyst, an organic-soluble palladium complex having a coordinating phosphine ligand; Alternatively, these protecting groups may be removed by the method of Tsuji described in Tetrahedron letters, *7*, 613 (1979). The Tsuji deprotection procedure utilizes an amine salt of formic acid and a mixture of a palladium compound and triphenylphosphine as the catalyst.

Compounds of formula IV can be prepared by following reaction scheme I:

REACTION SCHEME I

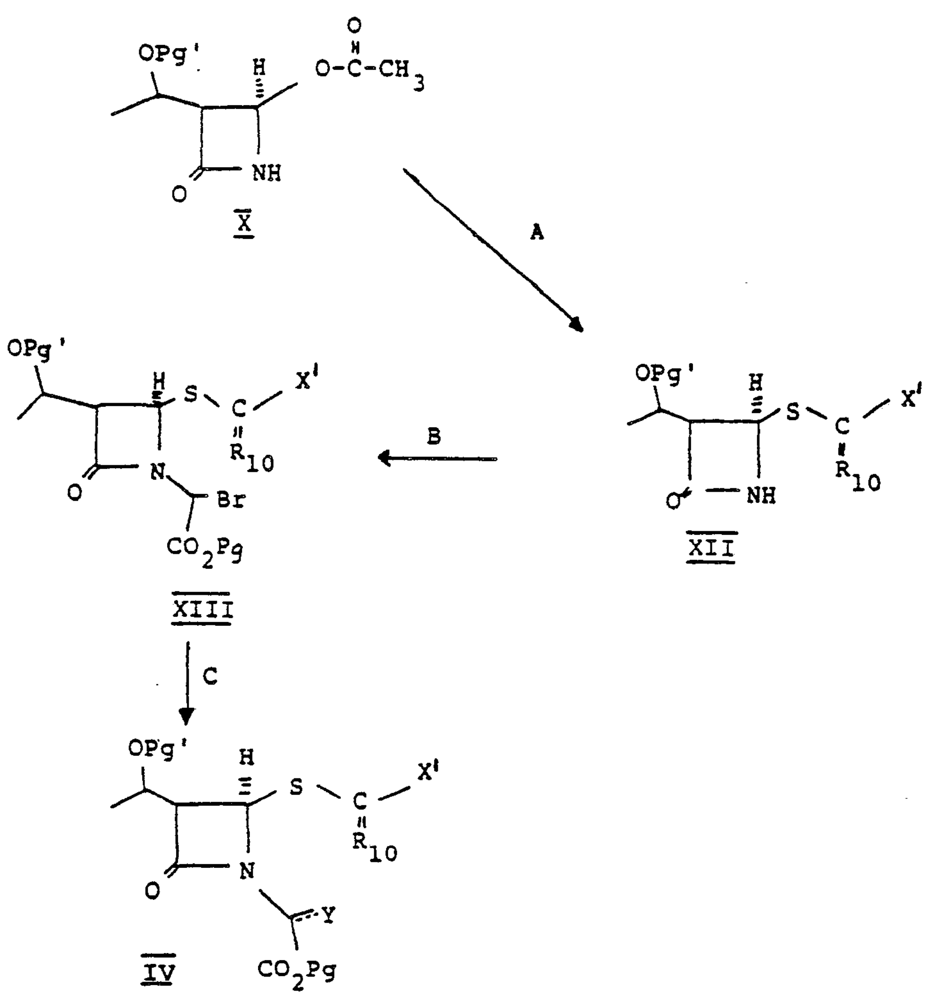

For purposes of illustration the reaction scheme shows the production of intermediates having the preferred 3-position substituent namely 1-protected-hydroxyethyl. It will be apparent that the reaction scheme may be applied equally to the preparation of intermediates in which the 3-position substituent is another protected-hydroxy loweralkyl group. The same consideration applies to reaction scheme II below.

In step A a 4-acetoxy-3-(protected-hydroxyethyl)acetidin-2-one of formula X in which Pg' is a removeable hydroxy protecting group is reacted with a compound of formula XI

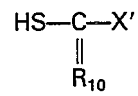

XI

in which $R_{10}$ and X' are as defined above, or a reactive derivative thereof, in which any functional groups are protected if necessary or desired.

The reaction of step A is generally conducted under basic condition, in aqueous media.

In the preferred procedure according to reaction scheme 1 compound XI has the formula XI'

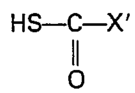

XI'

in which X' is as defined above, and the reaction mixture is formed, for example by adding a solution of the azetidinone of formula X in a water-miscible solvent, for example tetrahydrofuran or dioxane, to an aqueous solution of the thiolcarboxylic acid of formula XI' and sodium carbonate or sodium hydroxide. Generally the reaction between azetidinone X and thiolcarboxylic acid XI' can be carried out at a temperature in the range of 0°C to 50°C, preferably at room temperature, for 12 to 24 hours.

Step B involves a first stage in which a carboxy-protected 2-hydroxy acetic acid group is added to the nitrogen of the azetidinone ring in a compound of formula XII, and a second stage in which the hydroxy group of the 2-hydroxy acetic acid group is replaced by bromine or chlorine.

In the first stage the reaction is carried out by reacting the intermediate of formula XII with a glyoxylic ester of formula XIVa or its hemiacetal of formula XIVb

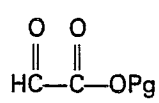

XIVa

or

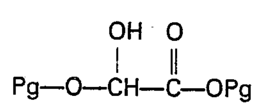

XIVb

in which Pg is a suitable easily removable protecting group, preferably one which can be removed under the same conditions as other protecting groups in the eventual cyclisation product. For this reason the allyl group is particularly preferred as it may later be removed concurrently with an allyloxycarbonyl group used for amino group protection. This reaction may be carried out in an organic solvent such as methylene chloride, chloroform or carbon tetrachloride and in the presence of a catalytic amount of an acid acceptor such as triethylamine or pyridine. The reaction time may be from 5 to 60 minutes and reaction temperature from 0° to 50°C, with room temperature being preferred.

The second stage of step B may be accomplished utilizing a halogenating agent, for example thionyl chloride, thionyl bromide, mesyl chloride, mesyl bromide, or a phosphorus oxyhalide especially the chloride, preferably in the presence of a base, preferably an organic base such as an aliphatic tertiary amine for example triethylamine, pyridine or collidine. Preferably the reaction is carried out in the presence of a suitable solvent such as methylene chloride, dioxane or tetrahydrofuran at a temperature in the range from −20°C to 0°C.

Step C involves the conversion of the halide intermediate of formula XIII into the intermediate of formula IV. This conversion is preferably accomplished by reaction of the halide intermediate with a suitable phosphine such as a triloweralkylphosphine, for example tri-*n*-butylphosphine or a triarylphosphine for example triphenylphosphine.

Triphenylphosphine is preferred for use in the present invention.

The reaction is preferably carried out in the presence of a suitable inert solvent such as dioxane, tetrahydrofuran or dimethylformamide. Depending upon the reactivity the reaction may be conducted with cooling or at elevated temperatures and is preferably carried out at room temperature.

The starting compounds of formula X may be obtained by methods known in the art, for example according to the teachings of European Patent Application Publications No. 3960 and 13662.

In a preferred method the compounds of formula X can be obtained by treating a penicillinate of the formula XX

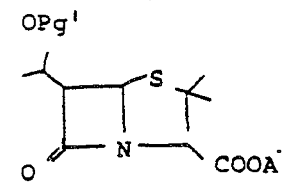

XX

in which Pg′ is as defined above and A is loweralkyl, with mercuric acetate in glacial acetic acid followed by separation of the resulting azetidinone intermediate of formula XXI

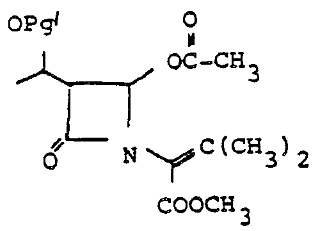

XXI

which intermediate is then treated with aqueous acetone and in the presence of potassium permanganate under cooling to give the desired compound of formula X.

Intermediates of formula XX are preparable for example following the procedure of preparation C paragraphs A to E in European Patent Application publication No. 0013662, starting from 6-β-amino penicillinic acid by:

1) first treating the acid in 2.5N sulfuric acid with sodium bromide, then at 0°C with sodium nitrate and bromine to form a mixture of 6,6-dibromo-penicillinic acid and 6-β-bromo-penicillinic acid,

2) treating the pencillinic acid mixture in dimethyl formamide at 0°C with finely powdered potassium carbonate and methyl iodide, and isolating pure methyl 6,6-dibromopenicillinate,

3) treating the ester from 2) in tetrahydrofuran, under nitrogen with cooling, with acetaldehyde in the presence of methylmagnesiumbromide to yield a mixture of methyl 6α-bromo-6β-(1-hydroxyethyl penicillinate and the corresponding 6α-(1-hydroxyethyl)-6β-bromo compound.

4) dehalogenation by treating with a 10% palladium on calcium carbonate catalyst under 2 atmospheres (100 KPa) hydrogen pressure; and

5) protection of the (1-hydroxyethyl group) by treatment with β,β,β-trichloroethylchloroformate in dry methylene chloride in the presence of pyridine and under a nitrogen atmosphere to give the methyl-6-(1-trichloroethoxycarbonyloxyethyl) penicillinate.

Compounds of formula XI can be prepared by methods known in the art.

For example compounds of formula XI′ can be prepared by converting the carboxy group of a carboxylic acid of formula XXIII

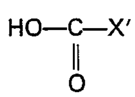

XXIII

in which any functional groups in X′ are protected if necessary or desired, to a thiolcarboxy group. This can be accomplished by dissolving the compound of formula XXIII in a suitable solvent, for example tetrahydrofuran or dioxane, treating it with isobutylchloroformate and an organic acid acceptor for example triethylamine or pyridine, followed by the addition of hydrogen sulfide gas. The reaction is preferably carried out at −20° to 0°C and for a period of 5 to 30 minutes.

The intermediates of formula XXIII can be produced by first protecting the amino group or groups in the group X′ of the appropriate carboxylic acid. By way of example, in the preparation of category (i) (a) and (i) (b) compounds the formula XXIII starting materials can be produced by first protecting the amino group

of an alpha amino carboxylic acid, for example of alpha amino carboxylic acid, for example of formula XXIII (a) and XXII (b)

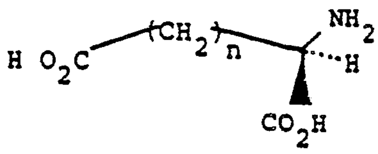

XXIII (a)

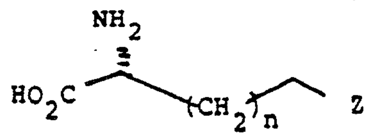

XXIII (b)

in which n and Z are as defined above to afford the corresponding alpha-N-protected carboxylic acid, for example of formula XXIV (a) and XXIV (b)

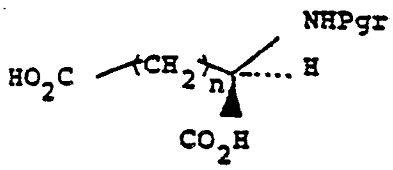

XXIV (a)

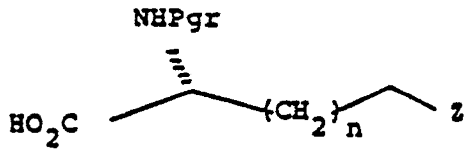

XXIV (b)

in which Pgr is a suitable readily removable amino protecting group, preferably allyloxycarbonyl. Usually the reaction is carried out in aqueous media to which a suitable inorganic base, such as sodium hydroxide has been added. The protecting group can then be added via a reactive derivative, for example the chloroformate.

In the case of the intermediates of formula XXIV and others used in the preparation of compounds of formula 1 (i) (a) the carboxylic group attached to the same carbon atom as the amino nitrogen (or group Q) should also be protected. This can be carried out by reacting the N-protected intermediate with allylbromide in the presence of an organic base e.g. triethylamine.

It will also be appreciated by the skilled man that functional groups contained in Z, may need to be protected and that such protection can be carried out in conventional manner.

Reactive derivatives of the compounds of formula XI include the metal salts e.g. alkali metal salts such as sodium or potassium salts.

An alternative method for the preparation of the compounds of formula IV involves the reaction of a suitably protected compound of formula XXIII or of a suitably protected mixed anhydride obtained by reacting a compound of formula XXIII with a suitable chloroformate, for example a loweralkyl chloroformate e.g. methylchloroformate or isobutylchloroformate in the presence of an acid acceptor, for example pyridine or triethylamine, with a silver salt of formula XV. The mixed anhydride is preferably prepared *in situ* in the reaction medium and reacted immediately after its preparation with the silver salt.

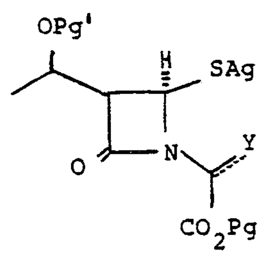

XV

in which Pg, Pg' and Y are as defined above.

This reaction is preferably carried out in an inert solvent for example tetrahydrofuran or ethyl ether preferably at temperatures of from −20°C to 0°C. Reaction times are preferably from 0.5 to 2 hours.

The silver salt of formula XV is preferably prepared by following the reaction scheme shown below

REACTION SCHEME II

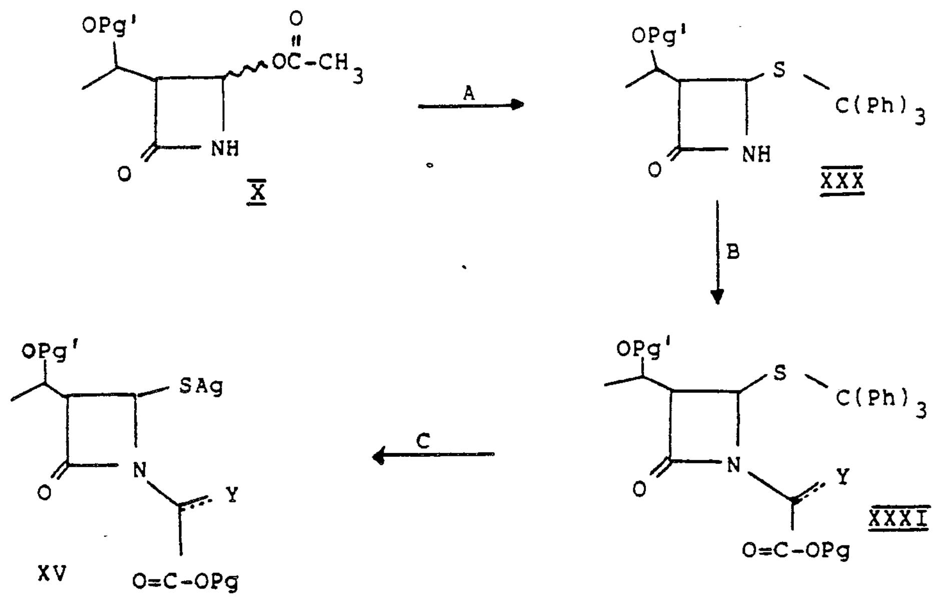


X XXX XXXI XV

Step IIA involves the conversion of a compound of formula X into a 4-triphenylmethylthio azetidinone intermediate of the formula XXX in which Pg′ is as defined above. This conversion is preferably carried out by reacting the compound of formula X with triphenylmethylthiol in the presence of an acid acceptor which may be inorganic, for example potassium or sodium carbonate or organic, for example triethylamine. The reaction is preferably carried out at 0° to 50°C (preferably at room temperature) and preferably in an organic solvent for example acetonitrile or pyridine. Reaction time depends upon the other reaction conditions utilized but usually is in the range of 2 to 24 hours.

Step IIB involves the conversion of the intermediate XXX into the phosphorane intermediate of formula XXXI, which is preferably accomplished using the same or similar procedure as described above for the conversion of an intermediate of formula XII to the intermediate of formula XIV.

Step IIC, involves the conversion of the intermediate of formula XXXI to the silver salt XV. This reaction is preferably carried out in a suitable organic solvent for example a halogenated hydrocarbon for example methylene chloride, using a reactive silver compound, usually a salt which is soluble in the selected solvent. Silver nitrate is the preferred reactive silver compound. An organic or inorganic base, for example aniline, pyridine, collidine or an alkali metal carbonate, is preferably included in the reaction mixture. Preferably the temperature range is from −20°C to 25°C. The reaction time depends upon the particular conditions and reagents, but is usually less than ½ hour.

Another process for the preparation of the compounds of formula I comprises reacting a compound having the general formula IV′

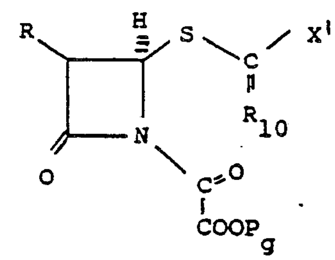

IV'

in which R, X, $R_{10}$ and Pg are as defined above any functional groups being protected if necessary or desired with a trivalent organophosphorus compound, preferably triethylphosphite, followed by removal of any protecting groups from the cyclised product before or after any desired separation of stereoisomers if a mixture of stereoisomers was subjected to cyclisation, and if necessary or desired subjecting the resulting compound to one or more of operations (i) to (iv) defined above in connection with the first mentioned method of preparation of the compounds of the invention.

In essence, this reaction is analogous to that described in our European Patent Application publication No. 58317.

In general the reaction can be carried out in an inert solvent for example aromatic hydrocarbons e.g. benzene, or toluene, aliphatic ethers e.g. diethylether or diisopropylether, cyclic ethers e.g. dioxane or tetrahydrofuran, and preferably halogenated hydrocarbons such as methylenechloride or chloroform.

The cyclisation reaction is preferably carried out in the temperature range 20°C to 80°C and preferably two molar equivalents of the phosphorus reagent is used.

The intermediates of formula IV′ can be obtained by reacting a compound of formula XII′

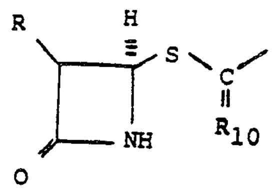

XII'

with an acid halide of the general formula

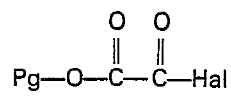

in which Pg is as defined above and Hal represents halogen e.g. chloro or bromo. The reaction can be carried out in an inert solvent in the presence of an organic base, preferably a tertiary amine and preferably also in the presence of an acid binding agent e.g. an alkaline earth metal carbonate such as calcium carbonate.

In addition certain compounds of this invention will form internal salts i.e. zwitterions, which may be obtained by neutralizing salts such as acid addition salts to the isoelectric point.

Salts may be converted in the usual manner into the free carboxy compounds.

Compounds of formula I can be prepared by stereospecific synthesis or from mixtures of stereoisomers and the resulting mixture of stereoisomers is separated if necessary or desired. The compounds of formula I can thus be prepared according to reaction scheme I and II using racemic starting materials. For instance, in the case of formula I(i)(a) compounds these may be prepared starting from racemic starting materials of formula XI which can be prepared using D, L mixtures of the compound shown in stereospecific form in formula XXIII(a). A desired isomer of formula I (or intermediate therefor) can be separated from a mxiture of stereoisomers as discussed herein.

Resulting mixtures of isomers can be separated into the individual isomers according to known methods. Diastereomeric mixtures, for example, can be separated by fractional crystallization absorption chromatography (column or thin layer) or other suitable separation methods. Resulting racemates can be resolved into the antipodes in the customary manner, for example, by forming a mixture of diastereomeric salts with optically active salt-forming reagents, separating the diastereomeric salts and converting the salts into the free compounds, or by fractional crystallization from optically active solvents.

## Preparation A

A. N-allyloxycarbonyl-D-methionine

To a solution of 15.00 g *D*-methionine in 25.2 ml 4N sodium hydroxide cooled to about 0°C add, with stirring, 15.60 ml allyl chloroformate and 41.49 ml 4N sodium hydroxide. After the addition is completed, stir the reaction for an additional ten minutes at 0°C and then allow to warm to room temperature for an additional 20 minutes. Wash the reaction mixture twice with ethyl ether. Separate aqueous layers, acidify to pH 2 with concentrated hydrochloric acid and then refrigerate for one hour. Extract the cooled solution three times with 100 ml portions of ethyl ether. Combine the ether extracts, dry them over anhydrous sodium sulfate and remove the solvents under vacuum to afford the title product having $[\alpha]_D^{26} = -6.5°$ ($CHCl_3$, c. = 0.4);

Mass spectra: $M^+$, 233; NMR: δ ($CDCl_3$) 2.13 ppm (s, 3H), 2.58 ppm (t, 2H).

B. N-allyloxycarbonyl-D-2-amino-4-methylthiothiolbutyric acid

To a solution of 10 g of the N-allyloxycarbonyl-D-methionine from paragraph A in 100 ml tetrahydrofuran and 7 ml pyridine at −15°C add a solution of 6.6 ml isobutylchloroformate in 10 ml tetrahydrofuran over a period of ten minutes. Stir the resulting suspension at about −15° to 10°C for one-half hour. Bubble hydrogen sulfide gas through the reaction mixture for fifteen minutes at about −10°C. Allow the reaction mixture to warm to room temperature until the evolution of carbon dioxide ceases and then dilute with 100 ml water. Acidify the solution with 25 ml 2N hydrochloric acid and then extract twice with 80 ml portions of ethyl acetate. Combine the extracts, wash twice with 100 ml portions of water, six times with 50 ml portions

of water, once with brine, and then dry over sodium sulfate. Remove the solvent by evaporation to afford the title compound having Mass spectra: $M^+ = 249$.

Preparation B

Utilize the procedure described in paragraph A of Preparation A and the appropriate amino acid, to produce the following intermediates:

N-Allyloxycarbonyl-S-Methyl-*D*-cysteine;
N-Allyloxycarbonyl-*D*-alanine;
N-Allyloxycarbonyl-*D*-serine;
N-Allyloxycarbonyl-*D*-homoserine;
N-Allyloxycarbonyl-*D*-histidine;
bis,N,*N*-Allyloxycarbonyl-*D*-lysine;
N-Allyloxycarbonyl-*D*-tyrosine;
N-Allyloxycarbonyl-*D*-phenylalanine;
N-Allyloxycarbonyl-*D*-tryptophan;

Preparation C

Convert the protected amino acids of Preparation B to the corresponding thioacids using the procedure of paragraph B of Preparation A to afford the following:

N-allyloxycarbonyl-*D*-2-amino-3-methylthiothiolpropionic acid;
N-allyloxycarbonyl-*D*-2-amino-thiolpropionic acid;
N-allyloxycarbonyl-*D*-2-amino-3-hydroxythiol-propionic acid;
N-allyloxycarbonyl-*D*-2-amino-4-thiolbutyric acid;
N-allyloxycarbonyl-*D*-2-amino-3-(4-imidazole)thiolpropionic acid;
bis-N,N-allyloxycarbonyl-*D*-2,6-diaminothiolcaproic acid;
N-allyloxycarbonyl-*D*-2-amino-3-*p*-hydroxyphenylthiolpropionic acid;
N-allyloxycarbonyl-*D*-2-amino-3-phenylthiolpropionic acid; and
N-allyloxycarbonyl-*D*-2-amino-3-indolethiolpropionic acid.

Preparation D

A. N-Allyloxycarbonyl-D-glutamic acid γ-allyl ester

Stir a solution of *D*-glutamic acid (6.0 g) in water (30 ml) and ethanol (10 ml) with copper carbonate (3.6 g) for 1 hour. To this mixture then add potassium bicarbonate (5.28 g) and allyl bromide (4.5 ml) and stir the mixture overnight at room temperature. Bubble excess hydrogen sulfide through the mixture, then filter and stir the filtrate vigorously with potassium bicarbonate (5.8 g) and allyl chloroformate (5.4 ml) for 1 hour. Wash the mixture with ethyl ether and acidify the aqueous phase with dilute mineral acid and extract with ethyl acetate. Dry the extract and evaporate. Purify the residual oil by chromatography on silica gel (100 g). Elute with chloroform:methanol:ammonia (7:1:0.2) to afford the title compound having $[\alpha]_D^{26} = +3.2°$ (c. = 4.8, chloroform)

B. N-Allyloxycarbonyl-D-glutamic α-thiolacid γ-allyl ester

The protected glutamic acid of paragraph A is converted to the title compound using the procedure of paragraph B of Preparation A.

Preparation E

N-Allyloxycarbonyl-L-glutamic acid α-allyl ester

A. Cool a solution of L-glutamic acid (6.58 g) in tetrahydrofuran (80 ml) and water (80 ml) containing sodium bicarbonate (11.36 g) to 4°C and stir while adding allyloxycarbonyl chloride (4.25 ml) dropwise during 45 minutes. Stir the mixture overnight at room temperature and then dilute with ethylacetate (100 ml). Separate the aqueous phase, acidify with mineral acid and extract with ethyl-acetate. Wash the extract with brine, dry and evaporate to give N-allyloxycarbonyl-L-glutamic acid, 8.9 g.

B. Treat a solution of N-allyloxycarbonyl-L-glutamic acid from Step A (8.8 g) in DMF (8 ml) and triethylamine (5.28 ml) with allyl bromide (3.3 ml). Stir the mixture overnight at room temperature and then dilute with water (150 ml) containing 10% sodium bicarbonate (40 ml). Wash the mixture with ether (2 × 100 ml), separate the aqueous layer, acidify with mineral acid and extract with ether. Wash the latter ether extract with brine, dry and evaporate to dryness to afford the title compound, 4.4 g, $[\alpha]_D^{26} = +0.3°$ (c = 27.7, $CHCl_3$]

Preparation F

Apply the reaction sequence described in Preparation E to D-glutamic acid, L- and D- aspartic acid and DL-α-amino adipic acid and obtain:

N-allyloxycarbonyl-D-glutamic acid α-allyl ester
N-allyloxycarbonyl-L-aspartic acid α-allyl ester
N-allyloxycarbonyl-D-aspartic acid α-allyl ester
N-allyloxycarbonyl-DL-α-aminoadipic acid α-allyl ester

Preparation G

N-Allyloxycarbonyl D-glutamic γ-Thiolacid α-allyl ester

Cool a solution of N-allyloxycarbonyl-D-glutamic acid α-allyl ester (2.7 g) and pyridine (0.8 g) in tetrahydrofuran (30 ml) to −10°C and stir while adding isobutylchloroformate (1.37 g) dropwise. Stir the mixture at −10° for 20 minutes and then bubble hydrogen sulfide gas therethrough for 20 minutes. Allow the mixture to warm up to room temperature and then dilute with water/10% sodium bicarbonate/ether. Separate the aqueous layer, acidify with mineral acid and extract with ethyl acetate. Wash the latter extract with brine, dry and evaporate to afford the title compound.

Preparation H

Convert the protected amino acids described in Preparation E and F to the following thiolacids, using the procedure of preparation G:

N-allyloxycarbonyl-L-glutamic γ-thiolacid α-allyl ester
N-allyloxycarbonyl-L-aspartic β-thiolacid α-allyl ester
N-allyloxycarbonyl-D-aspartic β-thiolacid α-allyl ester
N-allyloxycarbonyl-DL-α-aminoadipic δ-thiolacid α-allyl ester.

Preparation I

N-allyloxycarbonyl-isonipecotic acid

To a stirred solution of isonipecotic acid (5.0 g) in 4N sodium hydroxide (9.7 ml) and water (6 ml) add dropwise allylchloroformate (6.0 ml) and 4N sodium hydroxide (16 ml). Stir the reaction mixture for 1 hour and wash it with ether (50 ml). Acidify the aqueous phase with 1N hydrochloric acid and extract with ethyl acetate. Wash the organic layers with water, dry and evaporate to afford the title compound. M.S.: m/e 213 (M+)

Preparation J

N-allyloxycarbonyl nipecotic acid

A solution of nipecotic acid (5.0 g) in 4N sodium hydroxide (9.7 ml) and water (6 ml) was stirred while adding dropwise and simultaneously allylchloroformate (6.0 ml) and 4N sodium hydroxide (16 ml). The reaction mixture was then stirred for 1 hour and washed with ether (50 ml). The aqueous phase was acidified with 1N hydrochloric acid and extracted with ethyl acetate. The organic layers were then washed with water, dried and evaporated to afford the title compound. M.S.: m/e 213 (M+)

Preparation K

Repeat the procedure described above and obtain the following N-protected acids from the corresponding unprotected acids:

N-Allyloxycarbonyl Pipecolinic Acid
N-Allyloxycarbonyl D-Proline

Preparation L

(3S,4R,5R)-4-acetoxy-3-(1-trichloroethoxycarbonyloxyethyl)azetidin-2-one

To a solution of mercuric acetate (73.35 g) in glacial acetic acid (500 ml) at 80°C add in small lots methyl (5R,6S,8R)-6-(1-trichloroethoxycarbonyloxyethyl)penicillinate (50 g). After 2 hours, filter the mixture, dilute with two litres ethyl acetate, wash successively with water, 10% sodium bicarbonate solution, and brine and then dry and evaporate. Dissolve the resulting (3S,4R,5R)-1-[(2-methyl-1-methoxycarbonyl)prop-1-enyl]-3-(1-trichloroethoxycarbonyloxyethyl)-4-acetoxyazetidin-2-one in acetone (860 ml) and water (70 ml). Stir the solution and cool in an ice bath while adding potassium permanganate (23 g). After ½ hour dilute the solution with ethylacetate (500 ml), filter through celite, concentrate to 300 ml), dilute with an equal volume of ethylacetate and wash several times with water. Dry the organic layer and evaporate to afford the title compound having the following spectra:

NMR: δ 1.42 (d, J = 6 cps); 1.55 (d, J = 6 cps); 3.4 (dd, J = 2.8 cps); 4.76 (s), 5.86 (d, J = 1.5 cps); 5.90 (d, J = 3.0 cps).

Preparation M

(3R,4S,5R)-silver-3-(1′-trichloroethoxycarbonyloxy-1′-ethyl)-1-(allyl-2″-triphenylphosphoranylidene-2″-acetate)-2-azetidinone-4-thiolate

A. Stir overnight a solution of the title compound of Preparation L (50 g) in acetonitrile (750 ml) with potassium carbonate (39.6 g) and triphenylmercaptan (59.8 g) under argon. Filter the mixture and evaporate the filtrate to dryness. Chromatograph the resulting crude product on silica gel (540 g). Elute with 10% ethylacetate:hexane to obtain (3S,4R,5R)-3-(1′-trichloroethoxycarbonyloxyethyl)-4-tritylthio-azetidin-2-one.

B. Treat the product from Step A (55.9 g) in methylene chloride (600 ml) with allyl-glyoxylate-allyl-hemiacetal (17 g) and triethylene (1.0 g). After stirring for 1 hour, cool the solution in an ice bath and add mesyl bromide (62.96 g) in one lot and then add dropwise a solution of triethylamine (840 g) in methylene chloride (90 ml) while maintaining the reaction temperature below 2°C. After 1 hour filter the reaction

mixture through silica gel (300 g) eluting with 5% ethylacetate:methylene chloride; collect the eluates and evaporate. Dissolve the resulting bromo intermediate in dimethylformamide (300 ml). Add triphenylphosphine (30 g). Stir the reaction mixture for 15 hours at room temperature under an argon blanket. Dilute the solution with ethylacetate (500 ml), wash with 10% aqueous sodium bicarbonate, then with brine, dry over sodium sulfate and evaporate under reduced pressure. Chromatograph the resulting crude production on silica gel (1.5 kg). Elute with 20% ethylacetate:hexane to afford (3S,4R,5R)-3-(1′-trichloroethoxycarbonyloxyethyl)-1-(allyl-2″-triphenylphosphoranylidene-2″-acetate)-4-tritylthio-2-azetidinone.

C. Add to a mixture of 5.73 g (3S,4R,5R)-3-(1′-trichloroethoxycarbonyloxyethyl)-1-(allyl-2″-triphenylphosphoranylidene-2″-acetate)-4-tritylthio-2-azetidinone in 57 ml methanol sufficient methylene chloride to cause solution. Cool the solution to 0°C and add 0.92 ml pyridine, followed dropwise, over a 10 minute period, by a solution of 1.73 g silver nitrate in 8 ml water. After five minutes, pour the reaction mixture over 100 ml ice water. Separate the methylene chloride layer and extract the remaining water layer twice with 50 ml portions of ethyl acetate. Combine the methylene chloride layer and ethyl acetate layers, wash five times with 100 ml portions of cold water and then evaporate to give the title compound.

Preparation N

(4R,3S,5R,4′R)-3-(1-trichloroethoxycarbonyloxyethyl)-4-(N-allyloxycarbonyl-4′-amino-4′-allyloxycarbonylbutyroyl)-thio-1-[2″-(allyl-2″-triphenylphosphoranyl-acetate)]-azetidin-2-one

*Method A:*

Add isobutylchloroformate (0.15 ml) dropwise to a solution of N-allyloxycarbonyl-D-glutamic acid α-allyl ester (0.32 g) and pyridine (0.1 ml) in dry tetrahydrofuran (3 ml) at −20°C. After 15 minutes, add a solution of the title compound of Preparation M (0.8 g) in tetrahydrofuran (8 ml) to the reaction mixture which then stir at −20°C for 1 hour and then warm to room temperature. Dilute the mixture with ethyl acetate, filter, wash with 5% sodium bicarbonate, dry and evaporate. Chromatograph the crude reaction product on silica gel (20 g). Elute with 40% ethyl acetate/benzene to afford the title compound, 0.27 g, $[\alpha]_D^{26}$ = +2.7° (c=0.8, $CHCl_3$).

*Method B:*

To a solution of N-allyloxycarbonyl-D-glutamic thiolacid α-allyl ester (2.8 g) and sodium bicarbonate (0.84 g) in water (15 ml) and tetrahydrofuran (15 ml) add (3S,4R,5R)-4-acetoxy-3-(1-trichloroethoxycarbonyloxyethyl)-azetidin-2-one (3.2 g), stir the mixture overnight; dilute with water and extract with ethyl acetate to afford (3S, 4R, 5R)-4-(N-allyloxycarbonyl-D-glutamyl α-allyl ester) thio-3-(1-trichloroethoxycarbonyloxyethyl)-azetidin-2-one. Treat a solution of the latter product in methylene chloride (50 ml) with allylglyoxylate (1.3 g) and triethylamine (0.01 ml). After 30 minutes, cool the mixture to −10°C, add mesyl bromide (2.4 g) in one lot followed dropwise by a solution of triethylamine (1.5 g) in methylene chloride (10 ml) while maintaining the temperature below 5°C. After 30 minutes treat the reaction mixture with triphenylphosphine (4.0 g) in dimethylformamide (30 ml), then concentrate the solution to about 30 ml and allow it to stand at room temperture overnight. Dilute the reaction mixture with water, isolate the crude product by extraction with ethyl acetate and purify by chromatography on silica gel as described in Method A, to afford the title compound.

Using the appropriate producted amino acid/thiolacid of Preparation H and following the procedures described in Method A or Method B, obtain the following compounds:

(4R,3S,5R,4′S)-3-(1-trichloroethoxycarbonyloxyethyl)-4-(N-allyloxycarbonyl-4′-amino-4′-allyloxycarbonylbutyroyl)thio-1-[2″-(allyl-2″-triphenylphosphoranyl-acetate)]-azetidin-2-one: $[\alpha]_D^{26°}$ = +25.6° (c=4.7, $CHCl_3$).

(4R,3S,5R,3′R)-3-(1-trichloroethoxycarbonyloxyethyl)-4-(N-allyloxycarbonyl-3′-amino-3′-allyloxycarbonylpropionyl)-thio-1-[2″-(allyl-2″-triphenylphosphoranyl-acetate]-azetidin-2-one: $[\alpha]_D^{26°}$ = +9.5° (c=0.3, $CHCl_3$).

(4R,3S,5R,3′R)-3-(1-trichloroethoxycarbonyloxyethyl)-4-(N-allyloxycarbonyl-3′-amino-3′-allyloxycarbonylpropionyl)-thio-1-[2″-(allyl-2″-triphenyl-phosphoranyl-acetate)]azetidin-2-one: $[\alpha]_D^{26}$ = +36.8° (c.=0.3, $CHCl_3$).

(4R,3S,5R,5′RS)-3-(1-trichloroethoxycarbonyloxyethyl)-4-(N-allyloxycarbonyl-5′-amino-5′-allyloxycarbonylvaleryl)-thio-1-[2″-(allyl-2″-triphenylphosphoranyl-acetate)]-azetidin-2-one.

Preparation O

(4R,3S,5R)-3-(1-trichloroethoxycarbonyloxyethyl)-4-(N-allyloxycarbonyl-isonipecotinyl)thio-1-[2″-(allyl-2″-triphenylphosphoranyl-acetate)-azetidin-2-one

Cool to −20°C a solution of N-allyloxycarbonyl isonipecotic acid (1.68 g) in dry tetrahydrofuran (17 ml) containing pyridine (1.27 ml) and whilst stirring add dropwise a solution of methylchloroformate (0.61 ml) in tetrahydrofuran (3 ml) during 7 minutes. Stir the mixture for 20 minutes. Add to the reaction mixture over a period of 15 minutes 5.5 g of a solution of (3S,4R,5R) - silver - 3 - (1′ - trichloroethoxycarbonyloxyethyl) - 1 - (allyl - 2″ - triphenylphosphoranylidene - 2″ - acetate) - 2 - azetidinone - 4 - thiolate in tetrahydrofuran (20 ml). Stir the mixture at −15°C for 1 hour and then at room temperature for 45 minutes;

dilute the reaction mixture with 150 ml ethyl acetate and filter. Wash the filtrate with brine, dry and evaporate. Chromatograph the crude product on silica gel (45 g). Elute with 50% ethyl acetate-hexane to obtain the title compound. $[\alpha]_D$ + 17.8° (c=0.2, chloroform).

Preparation P

(4R,3S,5R)-3-(1-trichloroethoxycarbonyloxyethyl)-4-(N-allyloxycarbonyl-nipecotinyl)thio-1-[2″-(allyl-2″-triphenylphosphoranyl-acetate)]-azetidin-2-one

A solution of N-allyoxycarbonyl nipecotic acid (1.68 g) in dry tetrahydrofuran (17 ml) containing pyridine (1.27 ml) was cooled to −20°C and stirred while adding dropwise a solution of methylchloroformate (0.61 ml) in tetrahydrofuran (3 ml) during 7 minutes. The mixture was stirred for 20 minutes. A solution of (3S,4R,5R) - silver - 3 - (1′ - trichloroethoxycarbonyloxyethyl) - 1 - (allyl - 2″ - triphenylphosphoranylidene - 2″ - acetate) - 2 - azetidinone - 4 - thiolate (5.5 g) in tetrahydrofuran (20 ml) was then added dropwise to the reaction mixture during 15 minutes. The mixture was stirred at −15°C for 1 hour and at room temperature for 45 minutes, then diluted with 150 ml ethyl acetate and filtered. The filtrate was washed with brine, dried and evaporated. The crude product was chromatographed on silica gel (45 g). Elution with 50% ethyl acetate-hexane gave the title compound. M.S.: m/e 874 (M+).

Example 1

A. (3S,4R,5R,2′R)-3-(1-trichloroethoxycarbonyloxyethyl)-4-[2′-allyloxycarbonylamino-4′-methylthio-butyroyl)thio]-2-azetidinone

Dissolve *N*-allyloxycarbonyl-D-2-amino-4-methylthio-thiobutyric acid (prepared as described in Preparation A) in 20 ml water and 3.6 g sodium bicarbonate. To this solution add 13.4 g of 4-acetoxy-3-(1-trichloroethoxycarbonyloxyethyl)azetidin-2-one (prepared as described in Preparation L of this specification) dissolved in 100 ml portions of ethyl acetate. Stir the solution for 16 hours then extract with 100 ml portions of ethyl acetate. Combine the extracts, wash three times with 100 ml portions of water and dry over sodium sulfate. Evaporate the solvents to afford a residue which is purified by chromatography on 300 g silica gel to afford the title compound having $[\alpha]_D^{26}$ = +76.2° (c. = 0.6 $CHCl_3$);

Mass spectra, $M^+$ = 538; and

NMR: δ = 1.45 (d, J = 7 cps); 2.08; 2.5 (m), 3.38 (dd, J = 2,7 cps); 5.25 (d, J = 2 cps).

B. (3S,4R,5R,2′R)-1-(1″-allyloxycarbonyl-1″-hydroxymethyl)-3-(1-trichloroethoxycarbonyloxyethyl)-4-[(2′-allyloxycarbonylamino-4′-methylthiobutyroyl)thio]-2-azetidinone

Add to a solution of 20 g of (3S,4R,5R,2′R) - 3 - (1 - trichloroethoxycarbonyloxyethyl) - 4 - [2′ - allyloxycarbonyl - amino - 4′ - methylthiobutyroyl)thio] - 2 - azetidinone and 5.39 g 2-allyloxy-2-hydroxyacetic acid allyl ester in 100 ml methylene chloride a mixture of 1 ml of methylene chloride and 0.0513 ml triethylamine. Stir the resulting solution for fifteen minutes, whereupon thin layer chromatography indicates formation of the title product.

C. (3S,4R,5R,2′R)-1-(1″-allyloxycarbonyl-1″-bromomethyl)-3-(1-trichloroethoxycarbonyloxyethyl)-4-[2′-allyloxycarbonylamino-4′-methylthiobutyroyl)thio]-2-azetidinone

Cool a solution of (3S,4R,5R,2′R) - 1 - (1″ - allyloxycarbonyl - 1″ - hydroxymethyl) - 3 - (1 - trichloroethoxycarbonyloxyethyl) - 4 - [2′ - allyloxycarbonylamino - 4′ - methylthiobutyroyl)thio] - 2 - azetidinone in methylene chloride to about −10°C. Add thereto 5.9 g mesyl bromide in one portion with stirring. Then add a solution of 5.13 ml triethylamine in 10 ml methylene chloride dropwise over a period of about ten minutes. Chromatograph the reaction mixture on silica gel using 5:95 ethylacetate:methylene chloride to afford the title product.

D. Allyl-(3S,4R,5R,2′R)-3-(1-trichloroethoxycarbonyloxyethyl)-4-[2′-allyloxycarbonylamino-4′-methylthiobutyroyl)-thio]-2-azetidinon-1-yl-2″-triphenylphosphoranyl-2″-acetate

To a solution of (3S,4R,5R,2′R) - 1 - (1″ - allyloxycarbonyl - 1″ - bromomethyl) - 3 - (1 - trichloroethoxycarbonyloxyethyl) - 4 - [2′ - allyloxycarbonylamino - 4′ - methylthiobutyroyl)thio] - 2 - azetidinone in 100 ml dimethylformamide under a nitrogen atmosphere add 14.5 g triphenylphosphine. Stir the solution until homogeneous, then allow it to stand at room temperature for about 19 hours. Dilute the reaction mixture with 100 ml water and extract twice with 200 ml portions of ethyl acetate. Combine the extracts, wash six times with 50 ml portions of water and then once with brine. Evaporate the solvents to afford a residue which then chromatograph on 200 g silica gel, using 10:90 ethyl acetate:methylene chloride as eluant, to give the title compound as a colorless solid having $[\alpha]_D^{26}$ = +22,6° (c. = 0.3, $CHCl_3$); and

NMR: δ = 4.22 (dd, J = 2,8 cps); 5.61 (d, J = 2 cps).

E. Allyl-(5R,6S,8R,1′R)-6-(1-trichloroethoxycarbonyloxyethyl)-2-[N-allyloxycarbonyl-(1′-amino-3′-methylthio)-propyl]-2-penem-3-carboxylate

Reflux a solution of 16 g of allyl - (3S,4R,5R,2′R) - 3 - (1 - trichloroethoxycarbonyloxyethyl) - 4 - [(2′ - allyloxycarbonyl - amino - 4′ - methylthiobutyroyl)thio] - 2 - azetidinone - 1 - yl - 2″ - triphenyl-phosphoranyl - 2″ - acetate in 1.8 liters benzene under a nitrogen atmosphere for 34 hours. The remove

the solvent by evaporation and apply the residue as a benzene solution to a 150 g silica gel column. Chromatograph using 20:80 ethyl acetate:petroleum ether (30—60°) as eluant to afford the title compound having $[\alpha]_D^{26} = +84.1°$ (c. = 0.3, $CHCl_3$);

NMR: = 1.54 (d, J = 7 cps), 2.15 (s); 2.59 (m); δ 3.97 (dd, J = 1.5, 7 cps); 4.85 (s); 5.67 (d, J = 1.5 cps).

F. Allyl-(5R,6S,8R,1'R)-6-(1-hydroxyethyl)-2-[N-allyloxycarbonyl-(1'-amino-3'-methylthio)propyl]-2-penem-3-carboxylate

Cool a solution of allyl - (5R,6S,8R,1'R) - 6 - ( - trichloroethoxycarbonyloxyethyl) - 2 - [N - allyloxycarbonyl - (1' - amino - 3' - methylthio)propyl] - 2 - penem - 3 - carboxylate in 4 ml tetrahydrofuran to −15° to 20°C. Then add 1.2 ml glacial acetic acid and 1.2 ml water, followed by 0.4 g zinc in portions over a 3—1/2 hour period. When the reaction proceeds no further, add 20 ml ethyl acetate and filter the mixture. Wash the zinc residue three times with 20 ml portions of ethyl acetate. Add these washings to the filtrate and wash the solution successively with two 50 ml portions of brine, one 75 ml portion of 10% $NaHCO_3$ and two 50 ml portions of brine. After drying over anhydrous sodium sulfate, remove the solvents and chromatograph the residue on 7.6 g silica gel. Elute first with 15:85 hexane/chloroform and then chloroform to afford the title product as a white solid having $[\alpha]_D^{26} = +71.9°$, (c. = 0.3, $CHCl_3$);

Mass spectra: $M^+ = 442$;

NMR: δ = 1.34 (d, J = 7 cps); 2.13 (s), 2.56 (m); 3.73 (dd, J = 1.5, 7 cps); 5.57 (d, J = 1.5 cps).

G. (5R,6S,8R,1'R)-6-(1-hydroxyethyl)-2-[(1'amino-3'-methylthio)propyl]-2-penem-3-carboxylic acid

To a solution of 0.19 g of allyl - (5R,6S,8R,1'R) - 6 - (1 - hydroxyethyl) - 2 - [N - allyloxycarbonyl - (1' - amino - 3' - methylthio) - propyl] - 2 - penem - 3 - carboxylate in 1.9 ml ethyl acetate under nitrogen at room temperature, add 0.12 g triphenylphosphine, 0.072 g tetrakis (triphenylphosphine) palladium-(0) and 0.94 ml of 1.0 M 2-ethylhexanoic acid (in methylene chloride). After stirring one hour remove the solvent and dilute the residue with 10 ml water and 10 ml chloroform. Separate the aqueous layer, wash ten times with chloroform. Remove the residual chloroform to give the title product having $[\alpha]_D^{26} = +57.5°$, (c. = 0.3, $H_2O$);

IR: 5.65μ;

NMR: δ = 1.3 (d, J = 7 cps); 2.13 (s); 2.68 (m); 3.97 (dd, J = 1.5, 7 cps); 5.76 (d, J = 1.5 cps).

## Example 1A

A. Allyl-(3S,4R,5R,2'R)-3-(1-trichloroethoxycarbonyloxyethyl)-4-[2'-allyloxycarbonylamino-4'-methylthio-butyroylthio]-2-azetidin-1-yl-2"-triphenylphosphoranyl-2"-acetate

To a solution of N-allyloxycarbonyl-*D*-methionine in 9 ml of freshly distilled tetrahydrofuran cooled to about −15°C and maintained under a nitrogen atmosphere add 0.64 ml pyridine and 0.31 ml methyl chloroformate in 0.5 ml tetrahydrofuran. To the resultant N-allyloxycarbonyl-*D*-methionine mixed anhydride add dropwise over a ten minute period 2.08 ml of the (3S,4R,5R) - silver - 3 - (1' - trichloro-ethoxycarbonyloxyethyl) - 1 - (allyl - 2" - triphenylphosphoranylidine - 2" - acetate) - 2 - azetidinone - 4 - thiolate. Stir the mixture for 1—1/4 hour at −15° to −10°C, filter and then dilute with 100 ml ethyl acetate. Wash the mixture twice with 10% $NaHCO_3$, four times with 100 ml portions of water and dry over anhydrous sodium sulfate. Remove the solvents by evaporation to give a residue which is then chromatographed on 40 g silica gel. Elute with 30:70 ethyl acetate:hexane to afford the title compound as a white, puffy solid.

B. (5R,6S,8R,1'R)-6-(1-hydroxyethyl)-2-[1'-amino-3'-methylthio)propyl]-2-penem-3-carboxylic acid

Repeat the procedures detailed in Paragraphs E to G of Method A of Example 1 to afford the title compound.

## Examples 2—4

Repeat the procedures of Example 1 using appropriate starting materials to obtain the following compounds:

2. (5R,6S,8R,1'R)-6-(1-hydroxyethyl)-2-(1'-aminoethyl)-2-penem-3-carboxylic acid
   $[\alpha]_D^{26} = +61.1°$ (c. = 2.8, $H_2O$);
   IR: 5.62μ; NMR: δ = 1.27 (d, J = 6 cps); 1.52 (d, J = 7 cps); 3.92 (dd, J = 1.6,6 cps); 5.67 (d, J = 1.6 cps).

3. (5R,6S,8R,1'R)-6-(1-hydroxyethyl)-2[(1'-amino-3-carboxy)propyl]-2-penem-3-carboxylic acid
   IR: 5.64μ, 6.3μ;
   $[\alpha]_D = +67.5°$ (c = 1.6, $H_2O$);
   NMR: δ = 1.27 (d, J = 7 cps), 3.92 (dd, J = 7, 1.9 cps); 5.71 (d, J = 1.9 cps).

4. (5R,6S,8R,1'R)-6-(1-hydroxyethyl)-2-[(1'-amino-2'-methylthio)ethyl]-2-penem-3-carboxylic acid
   IR: 5.68μ;
   $[\alpha]_D = +7.2°$ (c = 0.25, $H_2O$);
   NMR δ = 1.35 (d, J = 6.5 cps); 2.2 (s); 3.1 (d, J = 7 cps); 4.0 (dd, J = 5, 1.8 cps); 5.8 (d, J = 1.8 cps);

Example 5

(5R,6S,8R,1'R)-6-(1-hydroxyethyl)-2-[N-acetimidoyl-(1'-amino-3'-methylthio)propyl]-2-penem-3-carboxylate, sodium salt

Treat a solution of (5R,6S,8R,1'R) - 6 - (1 - hydroxyethyl) - 2 - [(1' - amino - 3' - methylthio)propyl] - 2 - penem - 3 - carboxylic acid (0.3 g) in 0.5 M sodium 2-ethylhexanoate solution in water (2 ml) with ethylacetimidate (0.5 g) at 20°C. Stir the solution for 30 minutes and then chromatograph on Dowex 50 × 4, elute with water and lyophilize the fractions containing the title compound to give the title compound having: IR: 5.68μ.

Example 6

(5R,6S,8R,1'R)-6-(1'-hydroxyethyl)-2-[1'-guanidino-3'-methylthio)propyl]-2-penem-3-carboxylate, sodium salt

Treat a solution of the title compound from Example 1 (0.3 g) in aqueous 0.5 M sodium 2-ethylhexanoate (4 ml) with *S*-benzyl thiourea hydrochloride (0.3 g) for 1 hour. Isolate the title compound by reverse phase HPLC. IR: 5.68μ.

Utilizing suitable starting materials and substantially repeating the procedures described above produce compounds of formula A:

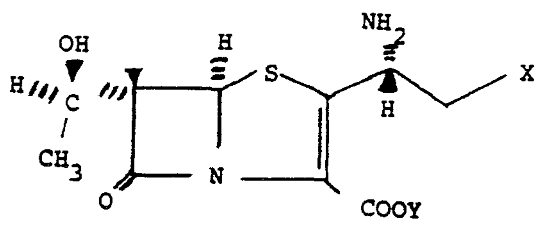

A

| X | Y |
|---|---|
| $-SCH_3$ | H |
| $-OH$ | H |
| $-COOH$ | H |
| H | H |
| $-CH_2OH$ | H |
| $NH_2$ | H |
| $-CH_2SCH_2CH_3$ | H |
| $-CH_2CH_2OH$ | H |
| $CH_2COOH$ | H |
| $CH_2NH_2$ | H |
| $CH_2CH_2COOH$ | H |
| $CH_2CH_2NH_2$ | H |
| $-CH_2CH_2SCH_3$ | H |
| $-CH_2CH_2SCH_2CH_3$ | H |
| $-CH_3$ | H |
| $-CH_2CH_3$ | H |
| $-CH_2CH_2CH_3$ | H |
| $-CH_2CH_2CH_2NH_2$ | H |
| $-CH_2CH_2CH_2OH$ | H |

| X | Y |
|---|---|
| $-CH_2CH_2CH_2COOH$ | H |
| $-CH_2CH_2CH_2SCH_3$ | H |
| $-CH_2CH_2CH_2SCH_2CH_3$ | H |
| $-(CH_2)_4-NH_2$ | H |
| $-(CH_2)_4-COOH$ | H |
| $-(CH_2)_4\ OH$ | H |
| $-(CH_2)_4-SCH_3$ | H |
| $-(CH_2)_3-CH_3$ | H |
| 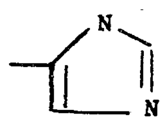 | H |
| 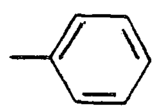 | H |
| 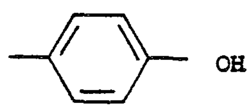 | H |
| 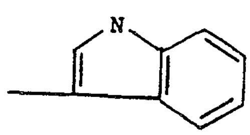 | H |
| $-CH_2SCH_3$ | Na |
| $-SCH_3$ | K |
| $-CH_2SCH_3$ | K |

Example 7

A. (5R,6S,8R,3′R) allyl-6-(1-trichloroethoxycarbonyloxyethyl)-2-(N-allyloxycarbonyl-3′-amino-3′-allyloxycarbonyl-propyl-2-penem-3-carboxylate

Reflux a solution of the title compound of Preparation I (0.26 g) in toluene (25 ml) for 3 hours under nitrogen. Evaporate the solution under reduced pressure and chromatograph the residue on silica gel (3 g). Elute the title compound (0.141 g) with ethyl acetate:hexane (2:6).

$[\alpha]_D^{26} = +76.9°$ (c = 2.6, $CHCl_3$)

M.S.: m/e 654 (M+)

NMR: δ = 1.47 (d, 3H, J = 6.6 cps), 1.64—2.42 (m, 2H), 3.87 (dd, 1H, J = 2.4, 9 cps), 5.57 (d, 1H, J = 2.4 cps).

B. (5R,6S,8R,3′R) allyl 6-(1-hydroxyethyl)-2-(N-allyloxycarbonyl-3′-amino-3′-allyloxycarbonyl)propyl-2-penem-3-carboxylate

Stir a solution of the title compound of step A of this Example (0.167 g) in tetrahydrofuran:water:acetic acid (2:0.5:0.5 ml) with zinc dust (0.2 g) until thin layer chromatography shows the reaction is complete. Dilute the mixture with ethyl acetate (30 ml), filter and wash the filtrate in succession with brine, aqueous sodium bicarbonate and brine, then dry over sodium sulfate and evaporate under reduced pressure. Chromatograph the residue on silica gel (5 g). Elute with 5% acetone-chloroform to afford the title compound, 0.14 g, m.p. 67—68°; $[\alpha]_D^{26} = +72.3°$ (c. = 3.25, $CHCl_3$);

IR: 5.62μ;
M.S.: m/e 480 (M+);
NMR: δ = 1.21 (d, 3H, J = 6 cps), 3.61 (dd, 1H, J = 6, 2 cps), 5.47 (d, 1H, J = 2 cps).

C. (5R,6S,8R,3′R) 6-(1-hydroxyethyl)-2-(3′-amino-3′-carboxy)propyl-2-penem-3-carboxylic acid monosodium salt

Add triphenyl phosphine (0.04 g) and tetrakis palladium triphenylphosphine (0.06 g) to a solution of the title compound of Step B (80 mg) in a mixture of 2.4 ml of 1M pyridinium formate and 0.24 ml 1M pyridine in methylene chloride under nitrogen. Stir the mixture for one hour and centrifuge. Wash the solid several times by resuspending and centrifuging, with methylene chloride/ethyl acetate. Then suspend the solid in 0.5 M sodium 2-ethyl hexanoate in ethyl acetate for 10 minutes and centrifuge and wash by centrifugation with ethyl acetate and ether to afford the title compound, 34 mg.
$[\alpha]_D^{26}$ = +105.8° (c = 4.3, water);
IR: 5.65μ;
NMR: δ = 1.27 (d, 3H, J = 6 cps), 3.83 (dd, 1H, J = 8, 2 cps), 5.61 (d, 1H, J = 2 cps).

Example 8

Repeat the procedure of Example 7 but using as starting material:

a) (4R,3S,5R,4′S)3-(1-trichloroethoxycarbonyloxyethyl)-4-(N-allyloxycarbonyl-4′-amino-4′-allyloxycarbonylbutyroyl)thio-1-[2″-allyl-2″-triphenylphosphoranyl acetate]azetidin-2-one;

b) (4R,3S,5R,3′R)3-(1-trichloroethoxycarbonyloxyethyl)-4-(N-allyloxycarbonyl-3′-amino-3′-allyloxycarbonyl propionyl)thio-1-(2″-allyl-2″-triphenylphosphoranyl acetate]azetidin-2-one;

c) (4R,3S,5R,3′S) 3-(1-trichloroethoxycarbonyloxyethyl)-4-(N-allyloxycarbonyl 3′-amino-3′-allyloxycarbonyl propionyl)thio-1-[2″-(allyl-2″-triphenylphosphoranyl acetate)]-azetidin-2-one;

d) (4R,3S,5R,5′RS) 3-(trichloroethoxycarbonyloxyethyl)-4-(N-allyloxycarbonyl-5′-amino-5′ allyloxycarbonyl-valeryl)-thio-1-[2″-allyl-2″-triphenylphosphoranyl acetate]-azetidin-2-one; and obtain respectively:

a) (5R,6S,8R,3′S) 6-(1-hydroxyethyl)-2-(3′-amino-3′-carboxy-propyl)-2-penem-3-carboxylic acid, monosodium salt
$[\alpha]_D$ = +110.9° (c = 2.3, water);
IR: 5.50μ;
NMR: δ = 1.25 (d, 3H, j = 6 cps), 3.79 (dd, 1H, J = 6, 2 cps), 5.53 (d, 1H, J = 2 cps);

b) (5R,6S,8R,2′R) 6-(1-hydroxyethyl)-2-(2′-amino-2′-carboxy-ethyl)-2-penem-3-carboxylic acid, monosodium salt
$[\alpha]_D^{26}$ = +124° (c = 0.36, water);
IR: 5.68μ;
NMR: δ = 1.23 (d, 3H, J = 6.8 cps), 3.83 (dd, 1H, J = 8, 2 cps) 5.58 (d, 1H, J = 2 cps).

c) (5R,6S,8R,2′S) 6-(1-hydroxyethyl)-2-(2′-amino-2′-carboxy ethyl)-2-penem-3-carboxylic acid, monosodium salt
$[\alpha]_D^{26}$ = +49.3° (c = 0.3, water)
NMR: δ = 1.26 (d, 3H, J = 7 cps), 3.88 (dd, 1H, J = 6, 2 cps), 5.61 (d, 1H, J = 2 cps).

d) (5R,6S,8R,4′RS) 6-(1-hydroxyethyl)-2-(4′-amino-4′-carboxybutyl)-2-penem-3-carboxylic acid.

Example 9

(5R,6S,8R,3′R) 6-(1-hydroxyethyl)-2-(N-acetimidoyl 3′-amino-3′-carboxypropyl)-2-penem-3-carboxylic acid, sodium salt

Stir a solution of (5R,6S,8R,3′R) 6-(1-hydroxyethyl)-2-(3′-amino-3′-carboxy)propyl-2-penem-3-carboxylic acid sodium salt (0.3 g) in water (3 ml) at 10°C with sodium bicarbonate (0.09 g) and ethylacetimidate (0.1 g) for 30 minutes. Lyophylize the solutions and purify the crude product by HPLC on $C_{18}$-silica gel to afford the title compound.
IR: 5.68μ

Example 10

(5R,6S,8R,3′R) 6-(1-hydroxyethyl)-2-(3′-guanidino-3′-carboxy-propyl)-2-penem-3-carboxylic acid, sodium salt

A solution of (5R,6S,8R,3′R) 6-(1-hydroxyethyl)-2-(3′-amino-3′-carboxy)propyl-2-penem-3-carboxylic acid, sodium salt from Example 1 (0.15 g) in water (1.5 ml) at 10°C is stirred with sodium bicarbonate (0.040 g) and S-benzyl thiourea hydrochloride (0.083 g) for 1 hour. The solution is then lyophylized and the title compound is obtained by chromatographing the crude lyophylized produce on $C_{18}$-silica gel.
IR: 5.68μ

Example 11

(5R,6S,8R)-6-(1-hydroxyethyl)-2-(4'-piperidinyl)-2-penem-3-carboxylic acid

A. Reflux a solution of (4R,3S,5R) - 3 - (1 - trichloroethoxycarbonyloxyethyl) - 4 - (N - allyloxycarbonylisonipecotinyl)thio - 1 - [2" - (allyl - 2" - triphenylphosphoranyl acetate)] - azetidin - 2 - one (1.57 g) in benzene (160 ml) under nitrogen for 12 days. Evaporate the solution to dryness and chromatograph the residue on silica gel (30 g). Elute with 30% ethyl acetate-hexane to obtain allyl (5R,6S,8R) 6 - (1-trichloroethoxycarbonyloxyethyl) - 2 - (4' - piperdinyl) - 2 - penem - 3 - carboxylate.

$[\alpha]_D$ +103.8° (c = 0.8, chloroform); M.S. m/e 596;

NMR: = 1.50 (d, 3H, J = 6 cps), 3.86 (dd, IH, J = 2,7 cps), 4.77 (S, 2H) 5.55 (d, IH, J = 2 cps)

B. Dissolve 0.27 g of the product from step A in tetrahydrofuran (3 ml) containing glacial acetic acid (0.85 ml) and water (0.85 ml). Cool the solution to −15°C and add zinc dust (0.25 g) in small lots during 2 hours while stirring the mixture vigorously. After a further 30 minutes filter the mixture, dilute the filtrate with brine and extract with ethyl acetate. Wash the extract with 5% aqueous sodium bicarbonate, then with brine, dry and evaporate under reduced pressure. Chromatograph the residue on silica gel (2 g). Elute with chloroform to obtain allyl (5R,6S,8R)-6-(1-hydroxyethyl)-2-(4'-piperidinyl)-2-penem-3-carboxylate.

$[\alpha]_D$ +103° (c = 2.0, $CHCl_3$); M.S.: m/e 422;

NMR: δ = 1.34 (d, 3H, J = 6 cps), 1.46 (m, 4H), 2.26 (m, 1H) 3.69 (dd, 1H, J = 2.7 cps), 5.56 (d, 1H, J = 2 cps)

C. Stir under nitrogen 0.06 g of a solution of the product from step B in methylene chloride (0.6 ml) containing tetrakis (triphenylphosphine) palladium (0.025 g), triphenylphosphine (0.04 g) and 1M 2-ethylhexanoic acid in methylene chloride (0.32 ml). A precipitate appears after a few minutes. Stir mixture for 1 hour and then shake with distilled water (10 ml). Wash the aqueous layer several times with chloroform and then lyophylize to afford the title compound.

$[\alpha]_D$ +96.8° (c = 0.23, water); IR 5.65;

NMR: δ = 1.3 (d, 3H, J = 6 cps), 1.82 (m, 4H), 3.83 (dd, 1H, J = 1.7, 7 cps), 5.61 (d, 1H, J = 1.7 cps)

Example 12

(5R,6S,8R,1'RS)-6-(1-hydroxyethyl)-2-(3'-piperidinyl)-2-penem-3-carboxylic acid

A solution of (4R,3S,5R) - 3 - (1 - trichloroethoxycarbonyloxyethyl) - 4 - (N - allyloxycarbonyl nipecotinyl)thio - 1 - [2" - (allyl - 2" - triphenylphosphoranyl acetate)]azetidin - 2 - one (1.57 g) in benzene (160 ml) was refluxed under nitrogen for 12 days. The solution was then evaporated to dryness and the residue was chromatographed on silica gel (30 g). Elution with 30% ethyl acetate-hexane afforded allyl (5R,6S,8R) - 6 - (1 - trichloroethoxycarbonyloxyethyl) - 2 - (3' - piperidinyl) - 2 - penem - 3 - carboxylate.

$[\alpha]_D$ +103.7° (c = 2.2, chloroform); M.S.: m/e 596

NMR: δ = 1.49 (d, J = 6.6 cps), 5.61 (d, J = 1.8 cps)

0.27 g of allyl (5R,6S,8R) - 6 - (1 - trichloroethoxycarbonyloxyethyl) - 2 - (3' - piperidinyl) - 2 - penem - 3 - carboxylate was dissolved in tetrahydrofuran (3 ml) containing glacial acetic acid (0.85 ml) and water (0.85 ml). The solution was cooled to −15°C and zinc dust (0.25 g) was added in small lots during 2 hours while the mixture was stirred vigorously. After a further 30 minutes the mixture was filtered, the filtrate was diluted with brine and extracted with ethyl acetate. The extract was then washed with 5% aqueous sodium bicarbonate, brine, dried and evaporated under reduced pressure. The residue was chromatographed on silica gel (2 g). Elution with chloroform afforded allyl (5R,6S,8R) - 6 - (1 - hydroxyethyl) - 2 - (3' - piperidinyl) - 2 - penem - 3 - carboxylate.

$[\alpha]_D$ +125° (c = 0.8, chloroform); IR = 5.60, 5.90μ; M.S.: m/e 422

NMR: δ = 1.31 (d, 3H, J = 6.2 cps), 3.64 (dd, 1H, J = 6, 1.8 cps)

0.06 g of a solution of allyl (5R,6S,8R) - 6 - (1 - hydroxyethyl) - 2 - (3' - piperidinyl) - 2 - penem - 3 - carboxylate in methylene chloride (0.6 ml) containing tetrakis (triphenylphosphine) palladium (0.025 g) triphenylphosphine (0.04 g) and 1M 2-ethylhexynoic acid in methylene chloride (0.32 ml) was stirred under nitrogen. A precipitate appeared after a few minutes. The mixture was stirred for 1 hr. and then shaken with distilled water (10 ml). The aqueous layer was washed several times with chloroform and then lyophylized to afford the title compound.

m.p. 172—73°C (dec.); IR: 5.65, 6.08—6.65;

NMR: δ(λ') 1.20 (d, 3H, J = cps), 3.78 (dd, 1H, J = 1.8, 6.6 cps), 5.58 (d, 1H, J = 1.8 cps)

Example 13

Repeat Preparation O and Example 11 using, as starting material in the repeat of Preparation instead of N-allyloxycarbonyl isonipecotic acid, the following:

(a) N-allyloxycarbonylpipecolinic acid;

(b) N-allyloxycarbonyl D-proline; to obtain respectively

(a') (5R,6S,8R,2'RS)-6-(1-hydroxyethyl)-2-2'-piperidinyl)-2-penem-3-carboxylic acid IR: 5.65μ;

(b') (5R,6S,8R,2'R)-6-(1-hydroxyethyl)-2-(2'-pyrrolidinyl)-2-penem-3-carboxylic acid IR: 5.65μ;

Example 14

(5R,6S,8R)-6-(hydroxyethyl)-2-(N-acetimidoyl-4'-piperidinyl)-2-penem-3-carboxylic acid

Treat with ethylacetimidate (0.2 g) a solution of the title compound of Example 11 (0.1 g) in water (1 ml) containing sodium bicarbonate (0.09 g). Stir the solution for 30 minutes and then chromatograph on Dowex 50×4. Elute with water, then lyophilize the eluates to obtain the title compound.

IR: 5.65μ

Example 15

(5R,6S,8R)-6-(1-hydroxyethyl)-2-(N-amidino-4'-piperidinyl)-2-penem-3-carboxylic acid

Stir with S-benzylthiourea hydrochloride (0.2 g), for one hour, a solution of the title compound of Example 11 (0.1 g) in water (1 ml) containing sodiuim bicarbonate (0.15 g). Isolate the title compound by reverse phase HPLC.

IR: 5.65μ

Following the procedures of the foregoing Examples and Intermediate preparations, and utilizing as intermediate the corresponding azacycloalkyl compounds, the following compounds of formula (XX) can be made:

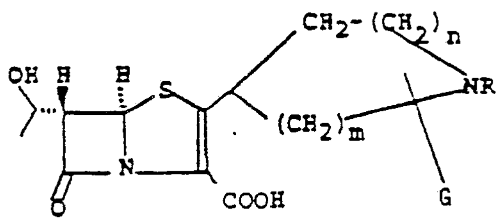

(XX)

| Stereochemical configuration | |
|---|---|
| | 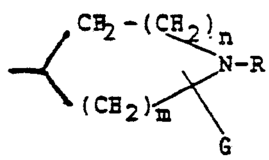 |
| 5R,6S,8R | 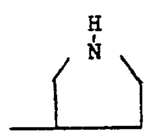 |
| 5R,6S,8R,2'R | 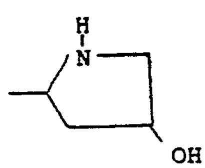 |
| 5R,6S,8R,2'R | 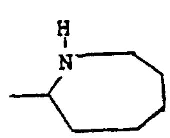 |
| 5R,6S,8R | 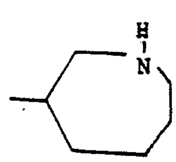 |

5R,6S,8R

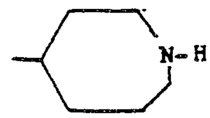

5R,6S,8R,2'R

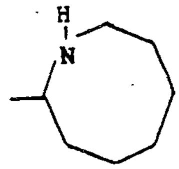

5R,6S,8R

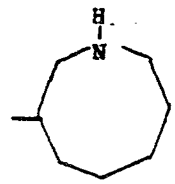

5R,6S,8R

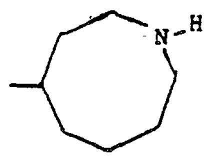

5R,6S,8R

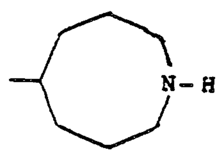

5R,6S,8R,2'R,6'R

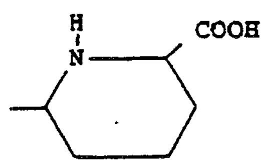

5R,6S,8R,2'R

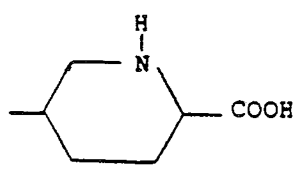

5R,6S,8R,2'R

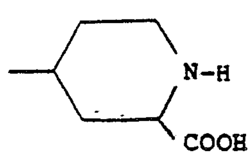

5R,6S,8R,2'R,7'R

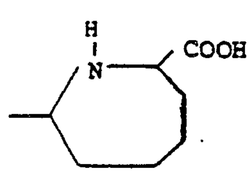

5R,6S,8R,2'R

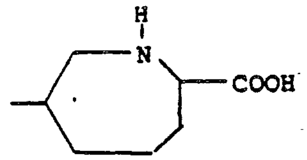

5R,6S,8R,2'R

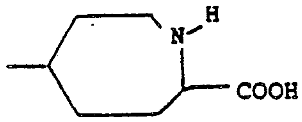

5R,6S,8R,2'R,8'R

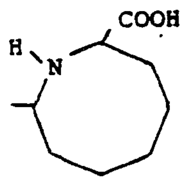

5R,6S,8R,2'R

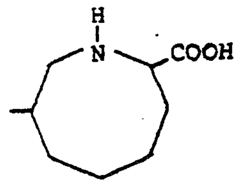

5R,6S,8R,2'R

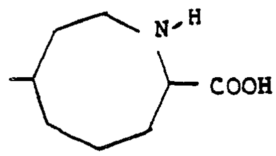

5R,6S,8R,2'R

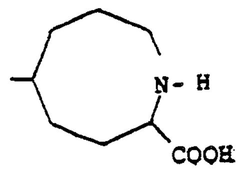

5R,6S,8R,2'R

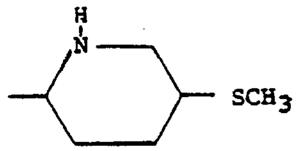

5R,6S,8R,2'R

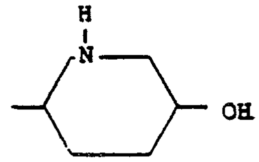

5R,6S,8R,2'R

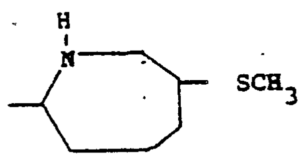

5R,6S,8R,2'R 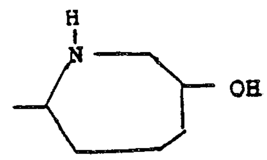

5R,6S,8R,2'R 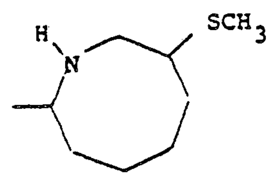

5R,6S,8R,2'R 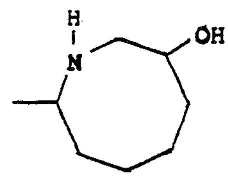

5R,6S,8R,2'R 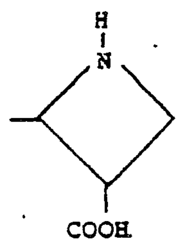

SR,6S,8R,2'R,4'R 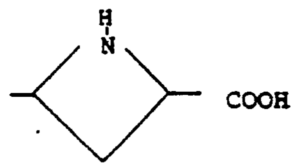

5R,6S,8R,2'R 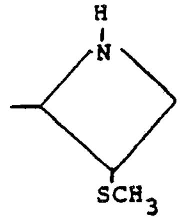

5R,6S,8R,2'R 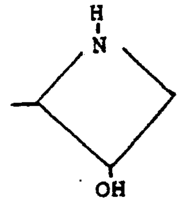

By way of example the following table gives mean *mic* values (μg/ml), for various of the compounds described in the above Examples, obtained from tests against a variety of gram+ and gram− organisms

| Compound | Mean MIC (μg/ml) | |
|---|---|---|
| | gram + | gram − |
| of Example 1 | 1.5 | 0.15 |
| of Example 2 | 14.3 | 0.29 |
| of Example 3 | 5.4 | 1.0 |
| of Example 4 | 1.33 | 0.18 |
| (5R,6S,8R,1'R)-6-(hydroxyethyl)-2-[(1'-amino-5' amino) pentyl]-2-penem -3-carboxylate | 38.6 | 0.75 |
| Example 7 | 5.0 | 2.7 |
| Example 8A | 17.3 | 3.4 |
| Example 8B | 22.4 | 11.7 |
| Example 8C | 46.1 | 11.0 |
| Example 11 | 3.7 | 0.1 |
| Example 12 | 1.96 | 0.038 |

In the following Examples 16—20, the active ingredient may be (5R,6S,8R,1'R) - 6 - (1 - hydroxyethyl) - 2 - [(1' - amino - 3' - methylthio)propyl] - 2 - penem - 3 - carboxylic acid ((5R,6S,8R,1'R) - 6 - (1 - hydroxyethyl) - 2 - [(1' - amino - 3' - carboxy) - propyl] - 2 - penem - 3 - carboxylic acid, (5R,6S,8R,3'R) - 6 - (1 - hydroxyethyl) - 2 - (3' - amino - 3' - carboxy - propyl) - 2 - penem - 3 - carboxylic acid or an equivalent amount of any of the other compounds of this invention.

Example 16

Capsules

| No. | Ingredient | mg/ capsule | mg/ capsule |
|---|---|---|---|
| 1. | Active ingredient | 250 | 500 |
| 2. | Lactose USP | 100 | 50 |
| 3. | Corn Starch, Food Grade | 50 | 43.5 |
| 4. | Microcrystalline Cellulose NF | 95 | 50 |
| 5. | Magnesium Stearate NF | 5 | 6.5 |
| | Total | 500 | 650 |

Method of Manufacture

Mix Items Nos. 1, 2, 3 and 4 in a suitable mixer for 10—15 minutes. Add Item No. 5 and mix for 1—3 minutes. Fill the mixture into suitable two-piece hard gelatin capsules using an encapsulating machine.

Example 17

Tablets

| No. | Ingredient | mg/ tablet | mg/ tablet |
|---|---|---|---|
| 1. | Active ingredient | 250 | 500 |
| 2. | Lactose USP | 57 | 114 |
| 3. | Corn Starch, Food Grade, as a 10% paste in Purified Water | 20 | 40 |
| 4. | Corn Starch, Food Grade | 18 | 39 |
| 5. | Magnesium Stearate NF | 5 | 7 |
| | Total | 350 | 700 |

Method of Manufacture

Mix Item Nos. 1 and 2 in a suitable mixer for 10—15 minutes. Granulate the mixture with Item No. 3. Pass the wet granulation through a coarse screen (e.g., 1/4″-6mm) if needed, and dry the wet granules. Mill the dried granules. Combine Item No. 4 and the dried granules and mix for 10—15 minutes. Add Item No. 5 and mix for 1—3 minutes. Compress the mixture to appropriate size and weight on a suitable tablet machine.

Example 18

Injectable Powder (per vial)

| | g/vial | g/vial |
|---|---|---|
| Active Ingredient | 0.5 | 1.0 |

Add sterile water for injection or bacteriostatic water for injection for reconstitution.

Example 19

Injectable Solution

| Ingredient | mg/ml | mg/ml |
|---|---|---|
| Active Ingredient | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection g.s.ad | 1.0 ml | 1.0 ml |

Method of Manufacture

1. Dissolve the parabens in a portion (85% of the final volume) of the water for injection at 65—70°C.
2. Cool to 25—35°C. Charge and dissolve the sodium bisulfite, disodium edetate and sodium sulfate.
3. Charge and dissolve the active ingredient.
4. Bring the solution to final volume by adding water for injection.

5. Filter the solution through 0.22μ membrane and fill into appropriate containers.
6. Terminally sterilize the units by autoclaving.

Example 20

Injectable Powder

| | g/vial |
|---|---|
| Active Ingredient | 1.0 |
| Sodium Citrate | 0.05 |

pH is adjusted to 6.2 using 0.1N citric acid solution.
Add sterile water for injection or bacteriostatic water for injection for reconstitution.

**Claims for the Contracting States: BE CH DE FR GB IT LI NL SE**

1. A compound having the formula I

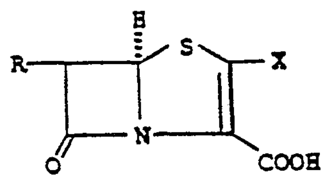

I

in which R is hydroxy $C_{1-6}$ alkyl, and
X is

(i) (a) 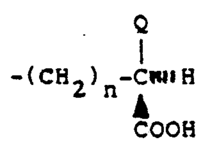 or

(b) 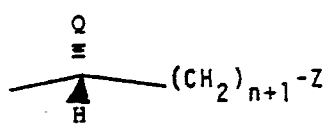

in which *n* is 0 to 4,
Q is $-NR_1R_2$ or

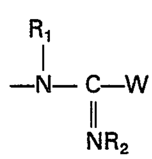

in which $R_1$ and $R_2$ are independently chosen from hydrogen, $C_{1-6}$ alkyl, $C_{2-6}$ alkenyl, heteroaryl being a heterocyclic group of aromatic character which contains 5 to 7 ring atoms of which 3 to 6 are carbon atoms and the remaining atoms are nitrogen, sulfur or oxygen atoms, phenyl, phenyl substituted by one or more groups chosen from chloro, bromo, fluoro, $C_{1-6}$ alkyl, hydroxy, nitro, amino, aminomethyl, mono-$C_{1-6}$ alkylamino, di-$C_{1-6}$ alkylamino, $C_{1-6}$ alkoxy, and carboxy, or $R_1$ is hydrogen and $R_2$ is acyl containing 2 to 18 carbon atoms and being the residue of a sulfonic acid or a carboxylic acid;
W is chosen from hydrogen, $C_{1-6}$ alkyl and amino; and

Z is chosen from hydrogen, 4-imidazolyl, 3-indolyl, phenyl, *p*-hydroxyphenyl, branched $C_{1-6}$ alkyl, $-COOR_1$, $-OR_1$, $-NR_1R_2$ or $-SR_1$, wherein $R_1$ and $R_2$ are as defined above; or X is

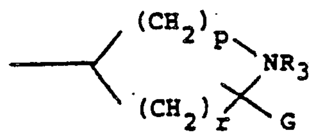

(ii)

in which *p* is 2 to 6, r is 0, 1, 2 or 3,

$R_3$ is hydrogen, $C_{1-6}$ alkyl, amidino or

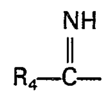

in which $R_4$ is hydrogen, or $C_1$—$C_3$ alkyl, and G is hydrogen, $C_{1-6}$ alkyl, $C_{1-6}$ alkoxy, amino, hydroxy, carboxy or $C_{1-6}$ alkylthio; and the pharmaceutically acceptable salts and metabolisable esters thereof.

2. A compound according to claim 1, in which R is 1-hydroxyethyl.

3. A compound according to claim 1 (i) (a) having the formula II(a)

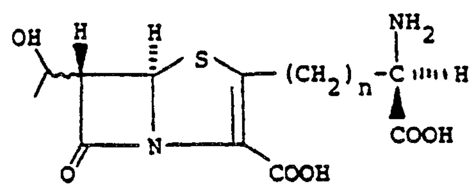

II(a)

in which *n* is as defined in claim 1, and the pharmaceutically acceptable salts and metabolisable esters thereof.

4. A compound according to claim 1 (i) (b) having the formula III

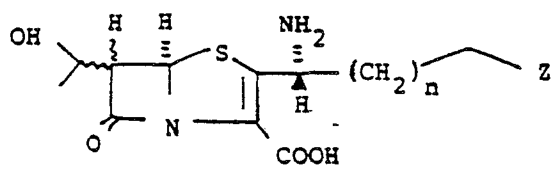

III

in which *n* is as defined in claim 1 and Z is chosen from hydrogen, carboxy, hydroxy, $C_{1-6}$ alkylthio, and the pharmaceutically acceptable salts and esters thereof.

5. A compound according to claim 1 (ii) in which *r* + *p* is 4, G is hydrogen or methyl, and $R_3$ is hydrogen, acetimidoyl or amidino and the pharmaceutically acceptable salts and metabolised esters thereof.

6. A compound according to claim 3, namely

(5R,6S,8R,1'R)-6-(1-hydroxyethyl)-2-(1'-amino-1'-carboxy)ethyl-2-penem-3-carboxylic acid,

(5R,6S,8R,2'R)-6-(1-hydroxyethyl)-2-(2'-amino-2'-carboxy)ethyl-3-penem-3-carboxylic acid,

(5R,6S,8R,3'R)-6-(1-hydroxyethyl)-2-(3'-amino-3'-carboxy)propyl-2-penem-3-carboxylic acid, and the pharmaceutically acceptable salts and metabolisable esters thereof.

7. A compound according to claim 1, namely

(5R,6S,8R,1'R)-6-(1-hydroxyethyl)-2-[(1'-amino-3'-carboxy)propyl]-2-penem-3-carboxylic acid,

(5R,6S,8R,1'R)-6-(1-hydroxyethyl)-2-[(1'-amino-3'-methylthio)propyl]-2-penem-3-carboxylic acid,

(5R,6S,8R,1'R)-6-(1-hydroxyethyl)-2-[(1'-amino-2'-methylthio)ethyl]-2-penem-3-carboxylic acid,

(5R,6S,8R,1'R)-6-(1-hydroxyethyl)-2-(1',5'-diaminopentyl)-2-penem-3-carboxylic acid,

(5R,6S,8R,1'R)-6-(1-hydroxyethyl)-2[(N-acetimidoyl-(1'-amino-3'-methylthio)propyl]-2-penem-3-carboxylic acid,

(5R,6S,8R,1'R)-6-(1-hydroxyethyl)-2-[(1'-guanidino-3'-methylthio)propyl]-2-penem-3-carboxylic acid and the pharmaceutically acceptable salts and metabolisable esters thereof.

8. A compound as defined in claim 1 (ii), namely
(5R,6S,8R)-6-(1-hydroxyethyl)-2-(4'-piperidinyl)-2-penem-3-carboxylic acid,
(5R,6S,8R)-6-(1-hydroxyethyl)-2-(3'-piperidinyl)-2-penem-3-carboxylic acid,
(5R,6S,8R,2'R)-6-(1-hydroxyethyl)-2-(2'-piperidinyl)-2-penem-3-carboxylic acid,
(5R,6S,8R,2'RS)-6-(1-hydroxyethyl)-2-(2'-piperidinyl)-2-penem-3-carboxylic acid,
(5R,6S,8R,2'R)-6-(1-hydroxyethyl)-2-(2'-pyrrolidinyl)-2-penem-3-carboxylic acid,
(5R,6S,8R)-6-(1-hydroxyethyl)-2-(N-acetimidoyl-4'-piperidinyl)-2-penem-3-carboxylic acid,
(5R,6S,8R)-6-(1-hydroxyethyl)-2-(N-amidino-4'-piperidinyl)-2-penem-3-carboxylic acid, and the pharmaceutically acceptable salts and esters thereof.

9. A pharmaceutical composition comprising a compound as defined in any one of the preceding claims, in admixture with a pharmaceutically acceptable carrier or excipient.

10. A process for the preparation of a compound of formula I as defined in claim 1, said process being characterized by:

A). intramolecular cyclisation of a compound of formula IV

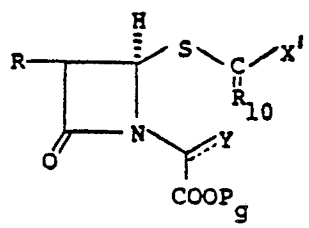

IV

in which R is as defined in claim 1, $R_{10}$ is oxygen or sulphur, $P_g$ is a carboxy protecting group, X' represents

a) 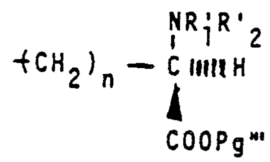

b) 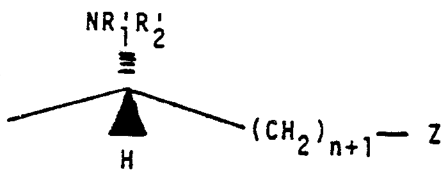

in which $R_1'$ and $R_2'$ represent respectively $R_1$ and $R_2$ as defined above except that they cannot simultaneously represent hydrogen or $R_1'$ corresponds to $R_1$ as defined above and $R_2'$ is an amino protecting group, Pg''' is a carboxy protecting group and any functional group in Z is protected of necessary or desired; or

c) 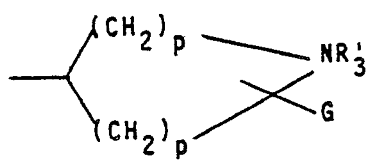

in which $R_3'$ is $C_{1-6}$ alkyl or an amino protecting group and any functional group in G is protected if necessary or desired, any other functional groups are protected if necessary or desired and Y is a phosphonio group being double bonded to the adjacent carbon atom or a phosphonato group with a single bond to the adjacent carbon atom the negative charge of which is compensated by the presence of a cation; or

B). reacting a compound of formula IV′

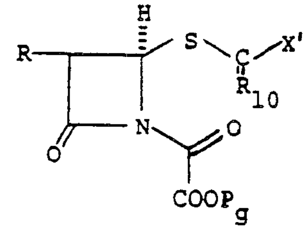

IV′

in which R, X′, $R_{10}$ and $P_g$ are as defined above, with a trivalent organophosphorus compound: followed by removal of any protecting groups before or after any necessary or desired separation of stereoisomers if a mixture of stereoisomers was subjected to cyclisation, and if necessary or desired subjecting the resulting compound to one or more of the following operations:

(i) converting a free acid group to a pharmaceutically acceptable salt or metabolisable ester group;
(ii) converting a salt to a pharmaceutically acceptable metabolisable ester or free acid;
(iii) introducing the grouping

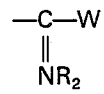

into an amine group represented by Q;

(iv) replacing the substituent $R_3'$ when it represents hydrogen by amidine or the group

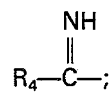

(v) separation of a mixture of enantiomers.

**Claims for the Contracting State: AT**

1. A process for the preparation of a compound of formula I

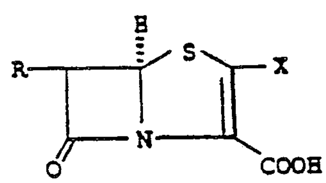

I

in which R is hydroxy $C_{1-6}$ alkyl, and

X is

(i) (a) 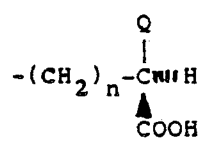 or

(b) 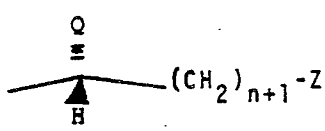

in which *n* is 0 or 4,

Q is $-NR_1R_2$ or

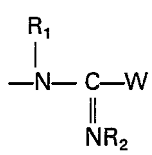

in which $R_1$ and $R_2$ are independently chosen from hydrogen, $C_{1-6}$ alkyl, $C_{2-6}$ alkenyl, heteroaryl, being a heterocyclic group of aromatic character which contains 5 to 7 ring atoms of which 3 to 6 are carbon atoms and the remaining ring atoms are nitrogen, sulfur or oxygen atoms, phenyl, phenyl substituted by one or more groups chosen from chloro, bromo, fluoro, $C_{1-6}$ alkyl, hydroxy, nitro, amino, aminomethyl, mono-$C_{1-6}$ alkylamino, di-$C_{1-6}$ alkylamino, $C_{1-6}$ alkoxy, and carboxy, or $R_1$ is hydrogen and $R_2$ is acyl containing 2 to 18 carbon atoms and being the residue of a sulfonic acid or a carboxylic acid;

W is chosen from hydrogen, $C_{1-6}$ alkyl and amino; and

Z is chosen from hydrogen, 4-imidazolyl, 3-indolyl, phenyl, *p*-hydroxyphenyl, branched $C_{1-6}$ alkyl, —$COOR_1$, —$OR_1$, —$NR_1R_2$ or —$SR_1$, wherein $R_1$ and $R_2$ are as defined above; or X is

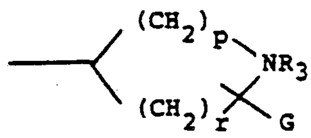

(ii)

in which *p* is 2 to 6,

*r* is 0, 1, 2 or 3,

$R_3$ is hydrogen, $C_{1-6}$ alkyl, amidino or

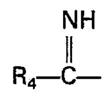

in which $R_4$ is hydrogen or $C_1$—$C_3$ alkyl, and G is hydrogen, $C_{1-6}$ alkyl, loweralkoxy, amino, hydroxy, carboxy or $C_{1-6}$ alkylthio; and the pharmaceutically acceptable salts and metabolisable esters thereof, said process being characterized by:

A) intramolecular cyclisation of a compound of formula IV

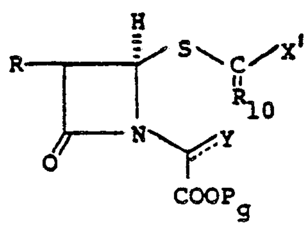

IV

in which R is as defined above, $R_{10}$ is oxygen or sulphur, $P_g$ is a carboxy protecting group, X' represents

a) 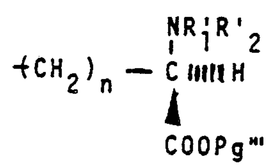

b) 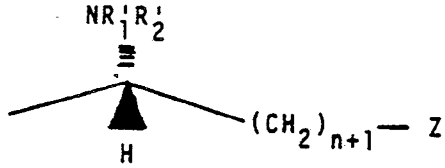

in which $R_1'$ and $R_2'$ represent respectively $R_1$ and $R_2$ as defined above except that they cannot simultaneously represent hydrogen or $R_1'$ corresponds to $R_1$ as defined above and $R_2'$ is an amino protecting group, Pg''' is a carboxy protecting group and any functional group in Z is protected of necessary or desired; or

c) 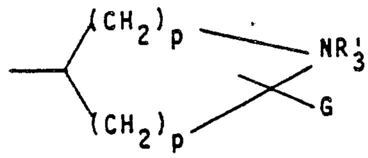

in which $R_3'$ is $C_{1-6}$ alkyl or an amino protecting group and any functional group in G is protected if necessary or desired, any other functional groups are protected if necessary or desired and Y is a phosphonio group being double bonded to the adjacent carbon atom or a phosphonato group with a single bond to the adjacent carbon atom the negative charge of which is compensated by the presence of a cation; or

B). reacting a compound of formula IV′

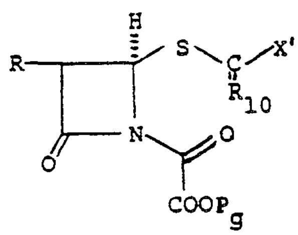

IV′

in which R, X′, $R_{10}$ and $P_g$ are as defined above, with a trivalent organophosphorus compound: followed by removal of any protecting groups before or after any necessary or desired separation of stereoisomers if a mixture of stereoisomers was subjected to cyclisation, and if necessary or desired subjecting the resulting compound to one or more of the following operations:

(i) converting a free acid group to a pharmaceutically acceptable salt or metabolisable ester group;

(ii) converting a salt to a pharmaceutically acceptable metabolisable ester or free acid;

(iii) introducing the grouping

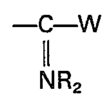

into an amine group represented by Q;

(iv) replacing the substituent $R_3'$ when it represents hydrogen by amidine or the group

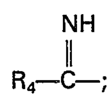

(v) separation of a mixture of enantiomers.

2. A process according to claim 1, in which a compound of formula I in which R is 1-hydroxyethyl is obtained.

3. A process according to claim 1 in which is obtained a compound having the formula II (a)

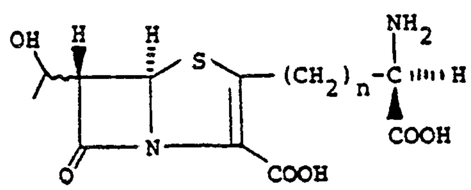

II(a)

in which *n* is as defined in claim 1, or a pharmaceutically acceptable salt or metabolisable ester thereof.

4. A process according to claim 1 in which is obtained a compound having the formula III

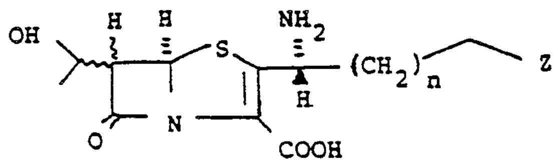

III

in which *n* is as defined in claim 1 and Z is chosen from hydrogen, carboxy, hydroxy, $C_{1-6}$ alkylthio or a pharmaceutically acceptable salt or metabolisable ester thereof.

5. A process according to claim 1 in which is obtained a compound of formula I (ii) in which $r+p$ is 4, G is hydrogen or methyl, and $R_3$ is hydrogen, acetimidoyl or amidino and the pharmaceutically acceptable salts and metabolised esters thereof.

6. A process according to claim 3, in which is obtained
(5R,6S,8R,1′R)-6-(1-hydroxyethyl)-2-(1′-amino-1′-carboxy)ethyl-2-penem-3-carboxylic acid,
(5R,6S,8R,2′R)-6-(1-hydroxyethyl)-2-(2′-amino-2′-carboxy)ethyl-3-penem-3-carboxylic acid,
(5R,6S,8R,3′R)-6-(1-hydroxyethyl)-2-(3′-amino-3′-carboxy)propyl-2-penem-3-carboxylic acid, or a pharmaceutically acceptable salt or metabolisable ester thereof.

7. A process according to claim 1, in which is obtained
(5R,6S,8R,1′R)-6-(1-hydroxyethyl)-2-[(1′-amino-3′-carboxy)propyl]-2-penem-3-carboxylic acid,
(5R,6S,8R,1′R)-6-(1-hydroxyethyl)-2-[(1′-amino-3′-methylthio)propyl)-2-penem-3-carboxylic acid,
(5R,6S,8R,1′R)-6-(1-hydroxyethyl)-2-[(1′-amino-2′-methylthio)ethyl]-2-penem-3-carboxylic acid,
(5R,6S,8R,1′R)-6-(1-hydroxyethyl)-2-(1′,5′-diaminopentyl)-2-penem-3-carboxylic acid,
(5R,6S,8R,1′R)-6-(1-hydroxyethyl)-2[(N′-acetimidoyl-[3′-methylthio)propyl]-2-penem-3-carboxylic acid,
(5R,6S,8R,1′R)-6-(1-hydroxyethyl)-2-[(1′-guanidino-3′-methylthio)propyl]-2-penem-3-carboxylic acid or a pharmaceutically acceptable salt or metabolisable ester thereof.

8. A process as defined in claim 1, in which is obtained
(5R,6S,8R)-6-(1-hydroxyethyl)-2-(4′-piperidinyl)-2-penem-3-carboxylic acid,
(5R,6S,8R)-6-(1-hydroxyethyl)-2-(3′-piperidinyl)-2-penem-3-carboxylic acid,
(5R,6S,8R,2′R)-6-(1-hydroxyethyl)-2-(2′-piperidinyl)-2-penem-3-carboxylic acid,
(5R,6S,8R,2′RS)-6-(1-hydroxyethyl)-2-(2′-piperidinyl-2-penem-3-carboxylic acid,
(5R,6S,8R,2′R)-6-(1-hydroxyethyl)-2-(2′-pyrrolidinyl)-2-penem-3-carboxylic acid,
(5R,6S,8R)-6-(1-hydroxyethyl)-2-(N-acetimidoyl-4′-piperidinyl)-2-penem-3-carboxylic acid,
(5R,6S,8R)-6-(1-hydroxyethyl)-2-(N-amidino-4′-piperidinyl)-2-penem-3-carboxylic acid, or a pharmaceutically acceptable salt or metabolisable ester thereof.

9. A process for preparing a pharmaceutical composition comprising mixing a compound of formula I as defined in any one of the preceding claims, with a pharmaceutically acceptable carrier or excipient.

10. A process for preparing a pharmaceutical composition comprising mixing a compound of formula I as defined in any of claims 1 to 8, when obtained by the process of any of claims 1 to 8, with a pharmaceutically acceptable carrier or excipient.

**Patentansprüche für die Vertragssaaten: BE CH DE FR GB IT LI NL SE**

1. Verbindung der Formel I

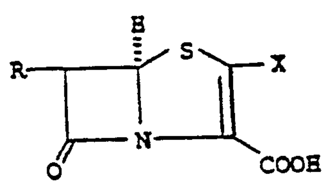

I

in welcher R Hydroxy—$C_{1-6}$—alkyl, und X

(i) (a) 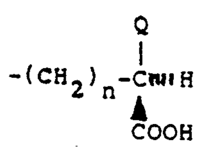 or

(b) 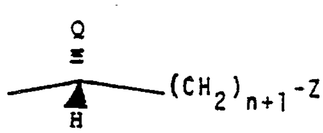

ist, in welchen *n* 0 bis 4 bedeutet,
*Q* für $—NR_1R_2$ oder

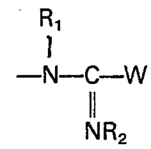

steht, in welchen $R_1$ und $R_2$ unabhängig voneinander aus Wasserstoff, $C_{1-6}$-Alkyl, $C_{2-6}$-Alkenyl, Heteroaryl, das eine heterocyclische Gruppe aromatischen Charakters ist, welche 5 bis 7 Ringatome enthält, von welchen 3 bis 6 Kohlenstoffatome und die verbleibenden Ringatome Stickstoff-, Schwefel- oder Sauerstoffatome sind, Phenyl, Phenyl substituiert durch eine oder mehrere Gruppen ausgewählt aus Chlor, Brom, Fluor, $C_{1-6}$—Alkyl, Hydroxy, Nitro, Amino, Aminomethyl, Mono—$C_{1-6}$—alkylamino, Di-$C_{1-6}$-alkylamino, $C_{1-6}$-Alkoxy und Carboxy, ausgewählt sind oder $R_1$ Wasserstoff und $R_2$ Acyl mit 2 bis 18 Kohlenstoffatomen und der Rest einer Sulfonsäure oder einer Karbonsäure ist;

W aus Wasserstoff, $C_{1-6}$-Alkyl und Amino und

Z aus Wasserstoff, 4-Imidazolyl, 3-Indolyl, Phenyl, p-Hydroxy-phenyl, verzweigtes $C_{1-6}$-Alkyl, $—COOR_1$, $—OR_1$, $—NR_1R_2$ oder $—SR_1$, ausgewählt ist, worin $R_1$ und $R_2$ obige Bedeutung haben; oder X

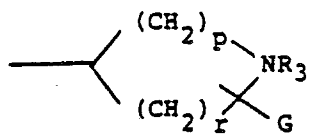

(ii)

ist, in welcher *p* 2 bis 6 bedeutet,
*r* 0, 1, 2 oder 3 ist,
$R_3$ Wasserstoff, $C_{1-6}$-Alkyl, Amidino oder

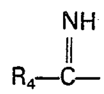

ist, in welcher
$R_4$ Wasserstoff oder $C_1$—$C_3$-Alkyl ist, und
G für Wasserstoff, $C_{1-6}$-Alkyl, Niederalkoxy, Amino, Hydroxy, Carboxy oder $C_{1-6}$-Alkylthio steht; und die pharmarzeutisch annehmbaren Salze und metabolisierbaren Ester davon.

2. Verbindung nach Anspruch 1, in welcher R 1-Hydroxyethyl ist.

3. Verbindung nach Anspruch 1 (i) (a), mit der Formel II (a)

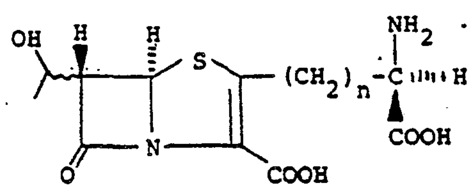

II (a)

in welcher *n* die in Anspruch 1 angegebene Bedeutung hat und die pharmazeutisch annehmbaren Salze oder metabolisierbaren Ester davon.

4. Verbindung nach Anspruch 1 (i) (b) mit der Formel III

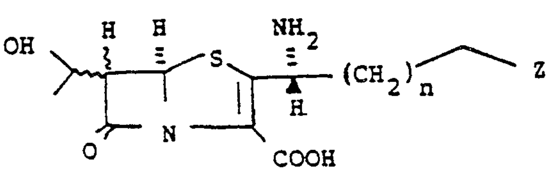

III

in welcher *n* die in Anspruch 1 angegebene Bedeutung hat und Z aus Wasserstoff, Carboxy, Hydroxy, $C_{1-6}$-Alkylthio ausgewählt ist und die pharmazeutisch annehmbaren Salze oder metabolisierbaren Ester davon.

5. Verbindung nach Anspruch 1 (ii), in welcher *r*+*p* 4 ist, G Wasserstoff oder Methyl bedeutet, und $R_3$

Wasserstoff, Acetimidoyl oder Amidino bedeutet, und die pharmazeutisch annehmbaren Salze und metabolisierbaren Ester davon.

6. Verbindung nach Anspruch 3, nämlich
(5R, 6S, 8R 1'R)-6-(1-Hydroxyethyl)-2-(1'amino-1'-carboxy)ethyl-2-penem-3-carbonsäure,
(5R, 6S, 8R, 2'R)-6-(1-Hydroxyethyl)-2-(2'-amino-2'-carboxy)-ethyl-3-penem-3-carbonsäure,
(5R, 6S, 8R, 3'R)-6-(1-Hydroxyethyl)-2-(3'-amino-3'-carboxy)propyl-2-penem-3-carbonsäure
und die pharmazeutisch annehmbaren Salze oder metabolisierbaren Ester davon.

7. Verbindung nach Anspruch 1, nämlich
(5R, 6S, 8R, 1'R)-6-(1-Hydroxyethyl)-2-[(1'-amino-3'-carboxy-propyl]-2-penem-3-carbonsäure,
(5R, 6S, 8R, 1'R)-6-(1-Hydroxyethyl)-2-[(1'-amino-3'-methylthio)propyl]-2-penem-3-carbonsäure,
(5R, 6S, 8R, 1'R)-6-(1-Hydroxyethyl)-2-[(1'-amino-2'-methylthio)ethyl]-2-penem-3-carbonsäure,
(5R, 6S, 8R, 1'R)-6-(1-Hydroxyethyl)-2-(1',5'-diaminopentyl)-2-penem-3-carbonsäure,
(5R, 6S, 8R, 1'R)-6-(1-Hydroxyethyl)-2-[(N-acetimidoyl-1'-amino-3'-methylthio)propyl]-2-penem-3-carbonsäure,
(5R, 6S, 8R, 1'R)-6-(1-Hydroxyethyl)-2-[(1'-guanidino-3'-methylthio)propyl]-2-penem-3-carbonsäure
und die pharmazeutisch annehmbaren Salze und metabolisierbaren Ester davon.

8. Verbindung nach Anspruch 1 (ii), nämlich
(5R, 6S, 8R)-6-(1-Hydroxyethyl)-2-(4'-piperidinyl)-2-penem-3-carbonsäure,
(5R, 6S, 8R)-6-(1-Hydroxyethyl)-2-(3'-piperidinyl)2-penem-3-carbonsäure,
(5R, 6S, 8R, 2'R)-6-(1-Hydroxyethyl)-2-(2'-piperidinyl)-2-penem-3-carbonsäure,
(5R, 6S, 8R, 2'RS)-6-(1-Hydroxyethyl)-2-(2'-piperidinyl)-2-penem-3-carbonsäure,
(5R, 6S, 8R, 2'R)-6-(1-Hydroxyethyl)-2-(2'-pyrrolidinyl)-2-penem-3-carbonsäure,
(5R, 6S, 8R)-6-(1-Hydroxyethyl)-2-(N-acetimidoyl-4'-piperidinyl)-2-penem-3-carbonsäure,
(5R, 6S, 8R)-6-(1-Hydroxyethyl)-2-(N-amidino-4'-piperidinyl)-2-penem-3-carbonsäure,
und die pharmazeutisch annehmbaren Salze und metabolisierbaren Ester davon.

9. Pharmazeutische Zusammensetzung aus einer der in einem der vorhergehenden Ansprüche definierten Verbindung in Mischung mit einem pharmazeutischen Träger oder Hilfsstoff.

10. Verfahren zur Herstellung einer Verbindung der in Anspruch 1 definierten Formel, welches Verfahren gekennzeichnet ist durch:

A) Intramolekulare Cyclisierung einer Verbindung der Formel IV

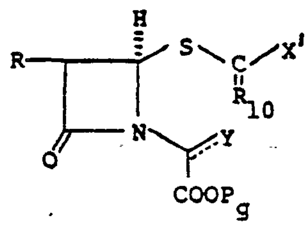

IV

in welcher R die in Anspruch 1 angegebene Bedeutung hat, $R_{10}$ Sauerstoff oder Schwefel ist, $P_g$ für eine Carboxyschutzgruppe steht, X'

a) 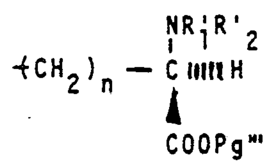

b) 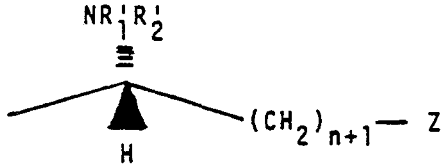

in welcher $R'_1$ und $R'_2$ die oben definierten Bedeutungen für $R_1$ bzw. $R_2$ haben, mit der Ausnahme, daß sie nicht gleichzeitig Wasserstoff sein können, oder $R'_1$ der oben definierten Bedeutung für $R_1$ entspricht und $R'_2$ eine Aminoschutzgruppe ist, $P_g'''$ eine Carboxyschutzgruppe ist und jede funktionelle Gruppe in Z, falls notwendig oder gewünscht, geschützt ist; oder

c) 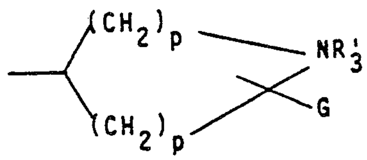

darstellt, in welcher $R'_3$ $C_{1-6}$-Alkyl oder eine Aminoschutzgruppe ist, und jede funktionelle Gruppe in G, falls notwendig oder erwünscht, geschützt ist, alle anderen funktionellen Gruppen, falls notwendig oder erwünscht, geschützt sind, und Y eine an das benachbarte Kohlenstoffatom doppelt gebundene Phosphoniogruppe oder eine Phosphonatgruppe mit einer Einfachbindung an das benachbarte Kohlenstoffatom ist, deren negative Ladung durch die Anwesenheit eines Kations ausgeglichen ist; oder

B) Umsetzung einer Verbindung der Formel IV'

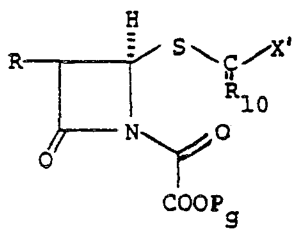

IV'

in welcher R, X', $R_{10}$ und $P_g$ obige Bedeutung haben, mit einer dreiwertigen Organophosphorverbindung; anschließender Entfernung der Schutzgruppen vor oder nach einer notwendigen oder erwünschten Trennung der Stereoisomeren, wenn eine Mischung von Stereoisomeren der Cyclisierung unterworfen wurde, und falls notwendig oder erwünscht, Unterwerfung der entstandenen Verbindungen einer oder mehrerer der folgenden Verfahrensschritte:

(i) Überführung einer freien Säuregruppe in ein pharmazeutisch annehmbares Salz oder eine metabolisierbare Estergruppe;

(ii) Überführung eines Salzes in einen pharmazeutisch annehmbaren metabolisierbaren Ester oder eine freie Säure;

(iii) Einführung der Gruppe

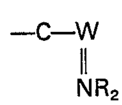

in eine durch Q dargestellte Aminogruppe;

(iv) Austausch des Substituenten $R'_3$, wenn er Wasserstoff darstellt, durch Amidin oder die Gruppe

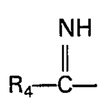 ;

(v) Trennung einer Mischung von Enantiomeren.

**Patentansprüche für den Vertragsstaat: AT**

1. Verfahren zur Herstellung einer Verbindung der Formel I

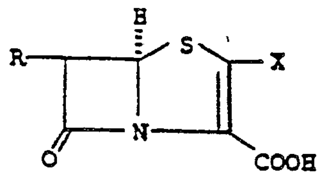

I

in welcher R Hydroxy-$C_{1-6}$-alkyl, und X

(i) (a) 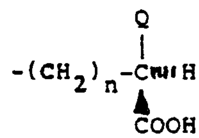 or

(b) 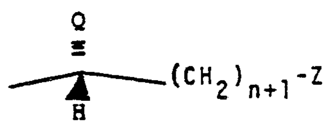

ist, in welchen *n* 0 bis 4 bedeutet,
*Q* für $—NR_1R_2$ oder

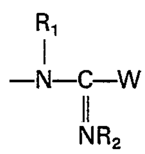

steht, in welchen $R_1$ und $R_2$ unabhängig voneinander aus Wasserstoff, $C_{1-6}$-Alkyl, $C_{2-6}$-Alkenyl, Heteroaryl, das eine heterocyclische Gruppe aromatischen Charakters ist, welche 5 bis 7 Ringatome enthält, von welchen 3 bis 6 Kohlenstoffatome und die verbleibenden Ringatome Stickstoff-, Schwefel- oder Sauerstoffatome sind, Phenyl, Phenyl, substituiert durch eine oder mehrere Gruppen ausgewählt aus Chlor, Brom, Fluor, $C_{1-6}$-Alkyl, Hydroxy, Nitro, Amino, Aminomethyl, Mono-$C_{1-6}$-alkylamino, Di-$C_{1-6}$-alkylamino, $C_{1-6}$-Alkoxy, und Carboxy, ausgewählt sind oder $R_1$ Wasserstoff und $R_2$ Acyl mit 2 bis 18 Kohlenstoffatomen und der Rest einer Sulfonsäure oder einer Karbonsäure ist;

W aus Wasserstoff, $C_{1-6}$-Alkyl und Amino und

Z aus Wasserstoff, 4-Imidazolyl, 3-Indolyl, Phenyl, p-Hydroxyphenyl, verzweigtes $C_{1-6}$-Alkyl, $—COOR_1$, $—OR_1$, $—NR_1R_2$ oder $—SR_1$, ausgewählt ist, worin $R_1$ und $R_2$ obige Bedeutung haben; oder X

(ii) 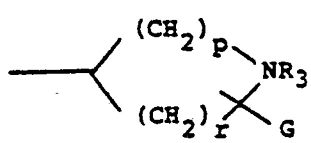

ist, in welcher *p* 2 bis 6 bedeutet,
*r* 0, 1, 2 oder 3 ist,
$R_3$ Wasserstoff, $C_{1-6}$-Alkyl, Amidino oder

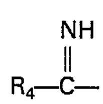

ist, in welcher

$R_4$ Wasserstoff oder $C_1$—$C_3$-Alkyl ist, und

G für Wasserstoff, $C_{1-6}$-Alkyl, Niederalkoxy, Amino, Hydroxy, Carboxy oder $C_{1-6}$-Alkylthio steht; und der pharmazeutisch annehmbaren Salze und metabolisierbaren Ester davon, welches Verfahren gekennzeichnet ist durch:

A) Intramolekulare Cyclisierung einer Verbindung der Formel IV

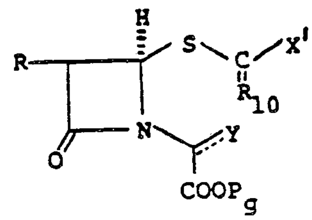

IV

in welcher R obige Beduetung hat, $R_{10}$ Sauerstoff oder Schwefel ist, $P_g$ für eine Carboxyschutzgruppe steht, X'

a)

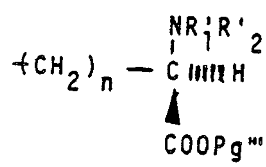

b)

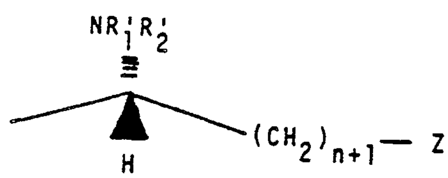

in welchen $R'_1$ und $R'_2$ die oben definierten Bedeutungen für $R_1$ bzw. $R_2$ haben, mit der Ausnahme, daß sie nicht gleichzeitig Wasserstoff sein können, oder $R'_1$ der oben definierten Bedeutung für $R_1$ entspricht und $R'_2$ eine Aminoschutzgruppe ist, $P_g'''$ eine Carboxyschutzgruppe ist und jede funktionelle Gruppe in Z, falls notwendig oder gewünscht, geschützt ist; oder

c)

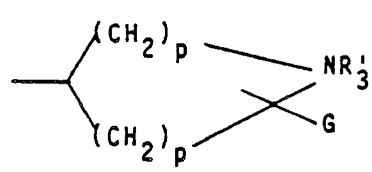

darstellt, in welcher $R'_3$ $C_{1-6}$-Alkyl oder eine Aminoschutzgruppe ist, und jede funktionelle Gruppe in G, falls notwendig oder erwünscht, geschützt ist, alle anderen funktionellen Gruppen, falls notwendig oder erwünscht, geschützt sind, und Y eine an das benachbarte Kohlenstoffatom doppelt gebundene Phosphoniogruppe oder eine Phosphonatgruppe mit einer Einfachbindung an das benachbarte Kohlenstoffatom ist, deren negative Ladung durch die Anwesenheit eines Kations ausgeglichen ist; oder

B) Umsetzung einer Verbindung der Formel IV'

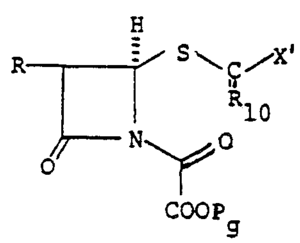

IV'

in welcher R, X′, $R_{10}$ und $P_g$ obige Bedeutung haben, mit einer dreiwertigen Organophosphorverbindung; anschließender Entfernung der Schutzgruppen vor oder nach einer notwendigen oder erwünschten Trennung der Stereoisomeren, wenn eine Mischung von Stereoisomeren der Cyclisierung unterworfen wurde, und falls notwendig oder erwünscht, Unterwerfung der entstandenen Verbindungen einer oder mehrerer der folgenden Verfahrensschritte:

(i) Überführung einer freien Säuregruppe in ein pharmazeutisch annehmbares Salz oder eine metabolisierbare Estergruppe;

(ii) Überführung eines Salzes in einen pharmazeutisch annehmbaren metabolisierbaren Ester oder eine freie Säure;

(iii) Einführung der Gruppe

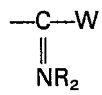

in eine durch Q dargestellte Aminogruppe;

(iv) Austausch des Substituenten $R'_3$, wenn er Wasserstoff darstellt, durch Amidin oder die Gruppe

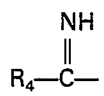 ;

(v) Trennung einer Mischung von Enantiomeren.

2. Verfahren nach Anspruch 1, in welchem eine Verbindung der Formel I, in welcher R 1-Hydroxyethyl ist, erhalten wird.

3. Verfahren nach Anspruch 1, in welchem eine Verbindung mit der Formel II (a)

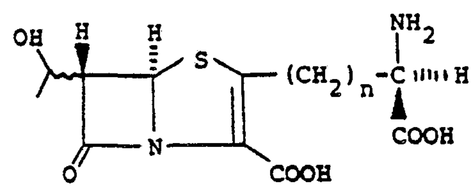

II (a)

in welcher *n* die in Anspruch 1 angegebene Bedeutung hat oder ein pharmazeutisch annehmbares Salz oder ein metabolisierbarer Ester davon erhalten wird.

4. Verfahren nach Anspruch 1, in welchem eine Verbindung mit der Formel III

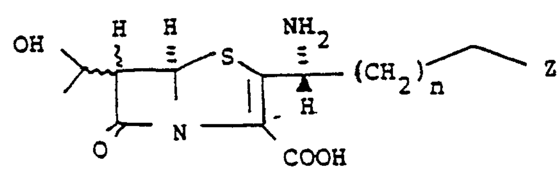

III

in welcher *n* die in Anspruch 1 angegebene Bedeutung hat und Z aus Wasserstoff, Carboxy, Hydroxy, $C_{1-6}$-Alkylthio ausgewählt ist oder ein pharmazeutisch annehmbares Salz oder metabolisierbare Ester davon, erhalten wird.

5. Verfahren nach Anspruch 1, in welchem eine Verbindung der Formel I (ii), in welcher *r*+*p* 4 ist, G Wasserstoff oder Methyl bedeutet, und $R_3$ Wasserstoff, Acetimidoyl oder Amidino bedeutet, und die pharmazeutisch annehmbaren Salze und metabolisierten Ester davon erhalten werden.

6. Verfahren nach Anspruch 3, in welchem

(5R, 6S, 8R 1′R)-6-(1-Hydroxyethyl)-2-(1′amino-1′-carboxy)ethyl-2-penem-3-carbonsäure,

(5R, 6S, 8R, 2′R)-6-(1-Hydroxyethyl)-2-(2′-amino-2′-carboxy)-ethyl-3-penem-3-carbonsäure,

(5R, 6S, 8R, 3′R)-6-(1-Hydroxyethyl)-2-(3′-amino-3′-carboxy)propyl-2-penem-3-carbonsäure oder ein pharmazeutisch annehmbares Salz oder metabolisierbarer Ester davon erhalten wird.

7. Verfahren nach Anspruch 1, in welchem

(5R, 6S, 8R, 1′R)-6-(1-Hydroxyethyl)-2-[(1′-amino-3′-carboxy)propyl]-2-penem-3-carbonsäure,

(5R, 6S, 8R, 1′R)-6-(1-Hydroxyethyl)-2-[(1′-amino-3′-methylthio)propyl]-2-penem-3-carbonsäure,

(5R, 6S, 8R, 1'R)-6-(1-Hydroxyethyl)-2-[(1'-amino-2'-methylthio)ethyl]-2-penem-3-carbonsäure,
(5R, 6S, 8R, 1'R)-6-(1-Hydroxyethyl)-2-(1',5'-diaminopentyl)-2-penem-3-carbonsäure,
(5R, 6S, 8R, 1'R)-6-(1-Hydroxyethyl)-2-[N-acetimidoyl-(3'-methylthio)propyl]-2-penem carbonsäure,
(5R, 6S, 8R, 1'R)-6-(1-Hydroxyethyl)-2-[(1'-guanidino-3'-methylthio)propyl]-2-penem-3-carbonsäure
oder ein pharmazeutisch annehmbares Salz oder metabolisierbarer Ester davon erhalten wird.

8. Verfahren nach Anspruch 1, in welchem
(5R, 6S, 8R)-6-(1-Hydroxyethyl)-2-(4'-piperidinyl)-2-penem-3-carbonsäure,
(5R, 6S, 8R)-6-(1-Hydroxyethyl)-2-(3'-piperidinyl)-2-penem-3-carbonsäure,
(5R, 6S, 8R, 2'R)-6-(1-Hydroxyethyl)-2-(2'-piperidinyl)-2-penem-3-carbonsäure,
(5R, 6S, 8R, 2'RS)-6-(1-Hydroxyethyl)-2-(2'-piperidinyl)-2-penem-3-carbonsäure,
(5R, 6S, 8R, 2'R)-6-(1-Hydroxyethyl)-2-(2'-pyrrolidinyl)-2-penem-3-carbonsäure,
(5R, 6S, 8R)-6-(1-Hydroxyethyl)-2-(N-acetimidoyl-4'-piperidinyl)-2-penem-3-carbonsäure,
(5R, 6S, 8R)-6-(1-Hydroxyethyl)-2-(N-amidino-4'-piperidinyl)-2-penem-3-carbonsäure,
oder ein pharmazeutisch annehmbares Salz oder metabolisierbarer Ester davon erhalten wird.

9. Verfahren zur Herstellung einer pharmazeutischen Zusammensetzung, dadurch gekennzeichnet, daß eine Verbindung der in einem der vorhergehenden Ansprüche definierten Formel I mit einem pharmazeutischen Träger oder Hilfsstoff vermischt wird.

10. Verfahren zur Herstellung einer pharmazeutischen Zusammensetzung, dadurch gekennzeichnet, daß eine Verbindung der in einem der Ansprüche 1 bis 8 definierten Formel I, wenn sie durch das Verfahren nach einem der Ansprüche 1 bis 8 erhalten wird, mit einem pharmazeutischen Träger oder Hilfsstoff vermischt wird.

**Revendications pour les Etats contractants: BE CH DE FR GB IT LI NL SE**

1. Composé ayant la formule I

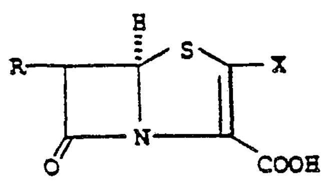

I

dans laquelle R est un hydroxyalcoyle $C_{1-6}$, et
X est

(i) (a) 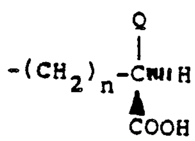 oder

(b) 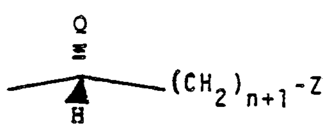

où *n* est 0 à 4,
Q est $—NR_1R_2$ ou

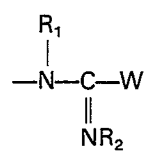

où $R_1$ et $R_2$ sont indépendamment choisis parmi l'hydrogène, un alcoyle $C_{1-6}$, un alkényle $C_{2-6}$, un hétéroaryle, étant un groupe hétérocyclique de caractère aromatique qui contient 5 à 7 atomes dans le noyau dont 3 à 6 sont des atomes de carbone et les atomes restants dans le noyau sont des atomes d'azote, de soufre ou d'oxygène, du phényle, du phényle substitué par un ou plusieurs groupes choisis parmi chloro, bromo, fluoro, alcoyle $C_{1-6}$, hydroxy, nitro, amino, aminométhyle, mono-alcoylamino $C_{1-6}$, di-alcoylamino $C_{1-6}$, alcoxy $C_{1-6}$ et carboxy ou bien $R_1$ est hydrogène et $R_2$ est acyle contenant 2 à 18 atomes

de carbone et étant le résidu d'un acide sulfonique ou d'un acide carboxylique; W est choisi parmi l'hydrogène, un alcoyle $C_{1-6}$ et un amino; et Z est choisi parmi l'hydrogène, 4-imidazolyle, 3-indolyle, phényle, *p*-hydroxyphényle, alcoyle $C_{1-6}$ ramifié, $—COOR_1$, $—OR_1$, $—NR_1R_2$ ou $—SR_1$, où $R_1$ et $R_2$ sont tels que définis ci-dessus; ou bien X est

(ii) 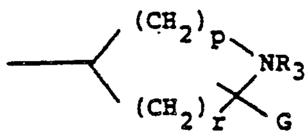

dans laquelle *p* est 2 à 6,
*r* est 0, 1, 2 ou 3,
$R_3$ est hydrogène, alcoyle $C_{1-6}$, amidino ou

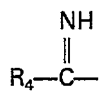

où
$R_4$ est hydrogène ou alcoyle $C_1—C_3$, et
G est hydrogène, alcoyle $C_{1-6}$, alcoxy $C_{1-6}$ amino, hydroxy, carboxy ou alkylthio $C_{1-6}$;
et les sels acceptables en pharmacie et esters métabolisables.

2. Composé selon la revendication 1 où R est 1-hydroxyéthyle.

3. Composé selon la revendication 1 (i) (a) ayant la formule II (a)

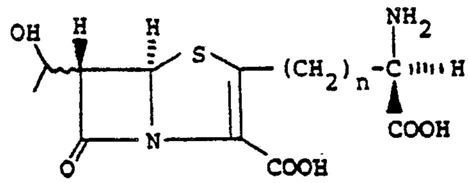

II (a)

où *n* est tel que défini à la revendication 1, et les sels et esters métabolisables acceptables en pharmacie.

4. Composé selon la revendication 1 (i) (b) ayant la formule III

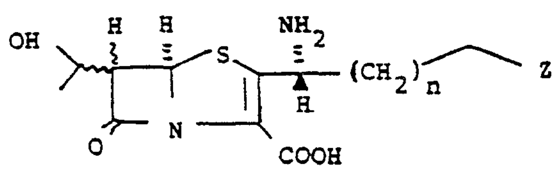

III

où *n* est tel que défini à la revendication 1 et Z est choisi parmi l'hydrogène, carboxy, hydroxy, alkylthio $C_{1-6}$ et les sels et esters acceptables en pharmacie.

5. Composé selon la revendication 1 (ii) où *r*+*p* est 4, G est hydrogène ou méthyle et $R_3$ est hydrogène, acétimidoyle ou amidino et les sels et esters métabolisables acceptables en pharmacie.

6. Composé selon la revendication 3, c'est-à-dire
l'acide (5R, 6S, 8R 1'R)-6-(1-hydroxyéthyl)-2-(1'-amino-1'-carboxy)èthyl-2-pénèm-3-carboxylique,
l'acide (5R, 6S, 8R, 2'R)-6-(1-hydroxyéthyl)-2-(2'-amino-2'-carboxy)èthyl-3-pénèm-3-carboxylique,
l'acide (5R, 6S, 8R, 3'R)-6-(1-hydroxyéthyl)-2-(3'-amino-3'-carboxy)propyl-2-pénèm-3-carboxylique,
et les sels et esters métabolisables acceptables en pharmacie.

7. Composé selon la revendication 1, c'est-à-dire
l'acide (5R, 6S, 8R, 1'R)-6-(1-hydroxyéthyl)-2-[(1'-amino-3'-carboxy)propyl]-2-pénèm-3-carboxylique,
l'acide (5R, 6S, 8R, 1'R)-6-(1-hydroxyéthyl)-2-[(1'-amino-3'-méthylthio)propyl]-2-pénèm-3-carboxylique,
l'acide (5R, 6S, 8R, 1'R)-6-(1-hydroxyéthyl)-2-[(1'-amino-2'-méthylthio)éthyl]-2-pénèm-3-carboxylique,
l'acide (5R, 6S, 8R, 1'R)-6-(1-hydroxyéthyl)-2-(1',5'-diaminopentyl)-2-pénèm-3-carboxylique,

l'acide (5R, 6S, 8R, 1'R)-6-(1-hydroxyéthyl)-2-[(N-acétimidoyl-(1'-amino-3'-méthylthio)propyl]-2-pénèm-3-carboxylique,

l'acide (5R, 6S, 8R, 1'R)-6-(1-hydroxyéthyl)-2-[(1'-guanidino-3'-methylthio)propyl]-2-pénèm-3-carboxylique,

et les sels et esters métabolisables acceptables en pharmacie.

8. Composé selon la revendication 1 (ii), c'est-à-dire

l'acide (5R, 6S, 8R)-6-(1-hydroxyéthyl)-2-(4'-pipéridinyl)-2-pénèm-3-carboxylique,

l'acide (5R, 6S, 8R)-6-(1-hydroxyéthyl)-2-(3'-pipéridinyl)-2-pénèm-3-carboxylique,

l'acide (5R, 6S, 8R, 2'R)-6-(1-hydroxyéthyl)-2-(2'-piperidinyl)-2-pénèm-3-carboxylique,

l'acide (5R, 6S, 8R, 2'RS)-6-(1-hydroxyéthyl)-2-(2'-pipéridinyl)-2-pénèm-3-carboxylique,

l'acide (5R, 6S, 8R, 2'R)-6-(1-hydroxyéthyl)-2-(2'-pyrrolidinyl)-2-pénèm-3-carboxylique,

l'acide (5R, 6S, 8R)-6-(1-hydroxyéthyl)-2-(N-acétimidoyl-4'-pipéridinyl)-2-pénèm-3-carboxylique,

l'acide (5R, 6S, 8R)-6-(1-hydroxyéthyl)-2-(N-amidino-4'-pipéridinyl)-2-pénèm-3-carboxylique,

et les sels et esters acceptables en pharmacie.

9. Composition pharmaceutique comprenant un composé tel que défini selon l'une quelconque des revendications précédentes, en mélange avec un véhicule ou excipient acceptable en pharmacie.

10. Procédé pour la préparation d'un composé de formule I tel que défini à la revendication 1, ledit procédé étant caractérisé par:

A) La cyclisation intramoléculaire d'un composé de formule IV

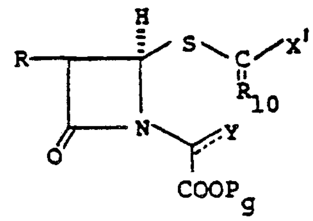

IV

où R est tel que dèfini à la revendication 1, $R_{10}$ est oxygène ou soufre, $P_g$ est un groupe carboxy protecteur, X' représente

a)

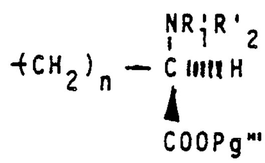

b)

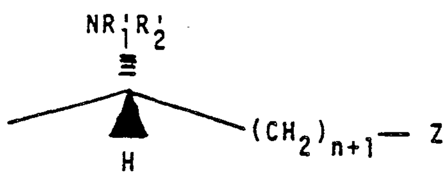

où $R'_1$ et $R'_2$ représentent respectivement $R_1$ et $R_2$ tels que dèfinis ci-dessus à l'exception qu'ils ne peuvent simultanément représenter de l'hydrogène ou bien $R'_1$ correspond à $R_1$ tel que défini ci-dessus et $R'_2$ est un groupe amino protecteur, Pg''' est un groupe carboxy protecteur et tout groupe fonctionnel dans Z est protégé si nécessaire ou souhaité; ou

c)

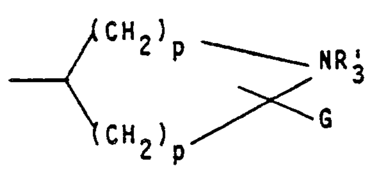

où $R'_3$ est alcoyle $C_{1-6}$ ou un groupe amino protecteur et tout groupe fonctionnel dans G est protégé si nécessaire ou souhaité, tous les autres groupes fonctionnels sont protégés si nécessaire ou souhaité et Y

est un groupe phosphonio étant doublement lié à l'atome de carbone adjacent ou un groupe phosphonato avec une simple liaison vers l'atome de carbone adjacent, dont la charge négative est compensée par la présence d'un cation; ou

B) La réaction d'un composé de formule IV'

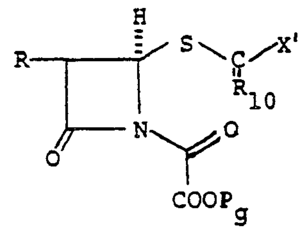

IV'

dans laquelle R, X', $R_{10}$ et $P_g$ sont tels que définis ci-dessus, avec un composé d'organophosphore trivalent;

avec ensuite enlèvement de tous les groupes protecteurs avant ou après toute séparation nécessaire ou souhaitée des stéréoisomères si un mélange de stéréoisomères a été soumis à une cyclisation et si nécessaire ou souhaité, en soumettant le composé résultant à une ou plusieurs des opérations suivantes:

(i) conversion d'un groupe acide libre en un sel ou un groupe ester métabolisable acceptable en pharmacie;

(ii) conversion d'un sel en un ester métabolisable ou acide libre acceptable en pharmacie;

(iii) introduction du groupement

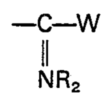

dans un groupe amine représenté par Q;

(iv) remplacement du substituant $R'_3$ lorsqu'il représente de l'hydrogène par une amidine ou le groupe

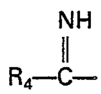 ;

(v) séparation d'un mélange d'énantiomères.

**Revendications pour l'Etat contractant: AT**

1. Procéde pour la préparatiion d'un composé de formule I

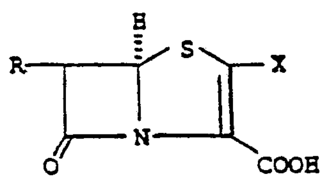

I

dans laquelle R est un hydroxyalcoyle $C_{1-6}$, et X est

(i) (a) 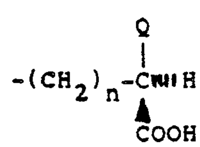 ou

(b) 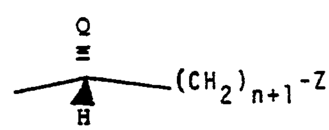

où *n* est 0 à 4,

Q est $—NR_1R_2$ ou

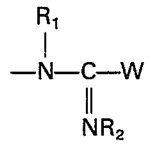

où $R_1$ et $R_2$ sont indépendamment choisis parmi l'hydrogène, un alcoyle $C_{1-6}$, un alkényle $C_{2-6}$, un hétéroaryle, étant un groupe hétérocyclique de caractère aromatique qui contient 5 à 7 atomes dans le noyau dont 3 à 6 sont des atomes de carbone et les atomes restants dans le noyau sont des atomes d'azote, de soufre ou d'oxygène, du phényle, du phényle substitué par un ou plusieurs groupes choisis parmi chloro, bromo, fluoro, alcoyle $C_{1-6}$, hydroxy, nitro, amino, aminométhyle, mono-alcoylamino $C_{1-6}$, di-alcoylamino $C_{1-6}$, alcoxy $C_{1-6}$ et carboxy ou bien $R_1$ est hydrogène et $R_2$ est acyle contenant 2 à 18 atomes de carbone et étant le résidu d'un acide sulfonique ou d'un acide carboxylique; W est choisi parmi l'hydrogène, un alcoyle $C_{1-6}$ et amino; et

Z est choisi parmi l'hydrogène, 4-imidazolyle, 3-indolyle, phényle, *p*-hydroxyphényle, alcoyle $C_{1-6}$ ramifié, $—COOR_1$, $—OR_1$, $—NR_1R_2$ ou $—SR_1$, où $R_1$ et $R_2$ sont tels que définis ci-dessus; ou bien X est

(ii) 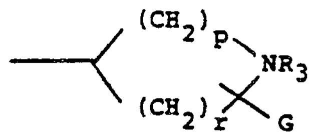

dans laquelle *p* est 2 à 6,

*r* est 0, 1, 2 ou 3,

$R_3$ est hydrogène, alcoyle $C_{1-6}$, amidino ou

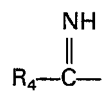

où

$R_4$ est hydrogène ou alcoyle $C_1—C_3$, et

G est hydrogène, alcoyle $C_{1-6}$, alcoxy inférieur, amino, hydroxy, carboxy ou alkylthio $C_{1-6}$;

et les sels acceptables en pharmacie et esters métabolisables, ledit procédé étant caractérisé par:

A) La cyclisation intramoléculaire d'un composé de formule IV

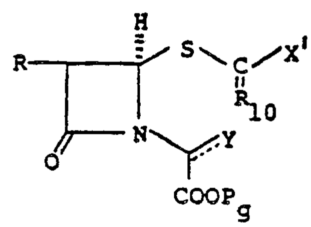

IV

où R est tel que dèfini ci-dessus, $R_{10}$ est oxygène ou soufre, $P_g$ est un groupe carboxy protecteur, X' représente

a) 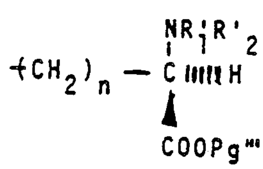

b) 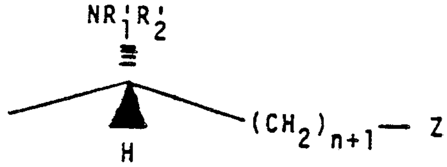

où $R'_1$ et $R'_2$ représentent respectivement $R_1$ et $R_2$ tels que dèfinis ci-dessus à l'exception qu'ils ne peuvent simultanément représenter de l'hydrogène ou bien $R'_1$ correspond à $R_1$ tel que défini ci-dessus et $R'_2$ est un groupe amino protecteur, Pg''' est un groupe carboxy protecteur et tout groupe fonctionnel de Z est protégé si nécessaire ou souhaité; ou

c) 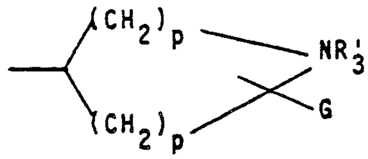

où $R'_3$ est alcoyle $C_{1-6}$ ou un groupe amino protecteur et tout groupe fonctionnel dans G est protégé si nécessaire ou souhaité, tous les autres groupes fonctionnels sont protégés si nécessaire ou souhaité et Y est un groupe phosphonio étant doublement lié à l'atome de carbone adjacent ou un groupe phosphoato avec une simple liaison vers l'atome de carbone adjacent, dont la charge négative est compensée par la présence d'un cation; ou

B) La réaction d'un composé de formule IV'

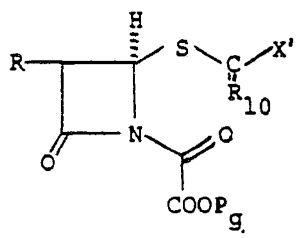

IV'

dans laquelle R, X', $R_{10}$ et $P_g$ sont tels que définis ci-dessus, avec un composé d'organophosphore trivalent;

avec ensuite enlèvement de tous les groupes protecteurs avant ou après toute séparation nécessaire ou souhaitée des stéréoisomères si un mélange de stéréoisomères a été soumis à une cyclisation et si nécessaire ou souhaité, en soumettant le composé résultant à une ou plusieurs des opérations suivantes:

(i) conversion d'un groupe acide libre en un sel ou un groupe ester métabolisable acceptable en pharmacie;

(ii) conversion d'un sel en un ester métabolisable ou acide libre acceptable en pharmacie;

(iii) introduction du groupement

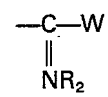

dans un groupe amine représenté par Q;

(iv) remplacement du substituant $R'_3$ lorsqu'il représente de l'hydrogène par une amidine ou le groupe

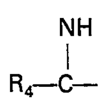 ;

(v) séparation d'un mélange d'énantiomères.

2. Procédé selon la revendication 1 où on obtient un composé de formule I dans lequel R est 1-hydroxyéthyle.

3. Procédé selon la revendication 1 où on obtient un composé ayant la formule II (a)

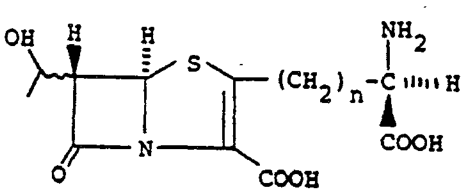

II (a)

où *n* est tel que défini à la revendication 1, ou un sel acceptable en pharmacie ou ester métabolisable.

4. Procédé selon la revendication 1 où on obtient un composé ayant la formule III

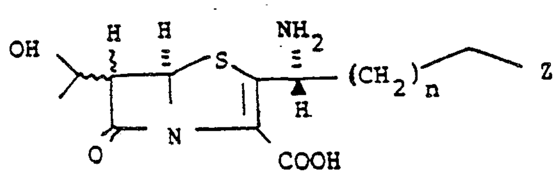

III

où *n* est tel que défini à la revendication 1 et Z est choisi parmi l'hydrogène, carboxy, hydroxy, alkylthio $C_{1-6}$ ou un sel ou ester métabolisable acceptable en pharmacie.

5. Procédé selon la revendication 1 où l'on obtient un composé de formule I (ii) où *r*+*p* est 4, G est hydrogène ou méthyle et $R_3$ est hydrogène, acétimidoyle ou amidino et les sels et esters métabolisables acceptables en pharmacie.

6. Procédé selon la revendication 3 où on obtient

l'acide (5R, 6S, 8R 1'R)-6-(1-hydroxyéthyl)-2-(1'-amino-1'-carboxy)èthyl-2-pénèm-3-carboxylique,

l'acide (5R, 6S, 8R, 2'R)-6-(1-hydroxyéthyl)-2-(2'-amino-2'-carboxy)èthyl-3-pénèm-3-carboxylique,

l'acide (5R, 6S, 8R, 3'R)-6-(1-hydroxyéthyl)-2-(3'-amino-3'-carboxy)propyl-2-pénèm-3-carboxylique,

ou un sel ou ester métabolisable acceptable en pharmacie.

7. Procédé selon la revendication 1 ou l'on obtient

l'acide (5R, 6S, 8R, 1'R)-6-(1-hydroxyéthyl)-2-[(1'-amino-3'-carboxy)propyl]-2-pénèm-3-carboxylique,

l'acide (5R, 6S, 8R, 1'R)-6-(1-hydroxyéthyl)-2-[(1'-amino-3'-méthylthio)propyl]-2-pénèm-3-carboxylique,

l'acide (5R, 6S, 8R, 1'R)-6-(1-hydroxyéthyl)-2-[(1'-amino-2'-méthylthio)éthyl]-2-pénèm-3-carboxylique,

l'acide (5R, 6S, 8R, 1'R)-6-(1-hydroxyéthyl)-2-(1',5'-diaminopentyl)-2-pénèm-3-carboxylique,

l'acide (5R, 6S, 8R, 1'R)-6-(1-hydroxyéthyl)-2-[(N-acétimidoyl-3'-méthylthio)propyl]-2-pénèm-3-carboxylique,

l'acide (5R, 6S, 8R, 1'R)-6-(1-hydroxyéthyl)-2-[(1'-guanidino-3'-methylthio)propyl]-2-pénèm-3-carboxylique,

ou un sel ou ester métabolisable acceptable en pharmacie.

8. Procédé selon la revendication 1 où l'on obtient

l'acide (5R, 6S, 8R)-6-(1-hydroxyéthyl)-2-(4'-pipéridinyl)-2-pénèm-3-carboxylique,

l'acide (5R, 6S, 8R)-6-(1-hydroxyéthyl)-2-(3'-pipéridinyl)-2-pénèm-3-carboxylique,

l'acide (5R, 6S, 8R, 2'R)-6-(1-hydroxyéthyl)-2-(2'-piperidinyl)-2-pénèm-3-carboxylique,

l'acide (5R, 6S, 8R, 2'RS)-6-(1-hydroxyéthyl)-2-(2'-pipéridinyl)-2-pénèm-3-carboxylique,

l'acide (5R, 6S, 8R, 2'R)-6-(1-hydroxyéthyl)-2-(2'-pyrrolidinyl)-2-pénèm-3-carboxylique,

l'acide (5R, 6S, 8R)-6-(1-hydroxyéthyl)-2-(N-acétimidoyl-4'-pipéridinyl)-2-pénèm-3-carboxylique,

l'acide (5R, 6S, 8R)-6-(1-hydroxyéthyl)-2-(N-amidino-4'-pipéridinyl)-2-pénèm-3-carboxylique,

ou un sel ou ester metabolisable acceptable en pharmacie.

9. Procédé de préparation d'une composition pharmaceutique consistant à mélanger un composé de formule I tel que défini selon l'une quelconque des revendications précedentes avec un véhicule ou excipient acceptable en pharmacie.

10. Procédé de préparation d'une composition pharmaceutique consistant à mélanger un composé de formule I tel que défini selon l'une quelconque des revendications 1 à 8, lorsqu'il est obtenu par le procédé selon l'une quelconque des revendications 1 à 8, avec un véhicule ou excipient acceptable en pharmacie.